# EUROPEAN PATENT APPLICATION

(11) **EP 2 319 576 A1**
(43) Date of publication of application: **11.05.2011**
(21) Application number: 10014995.4
(22) Date of filing: 13.03.2009
(51) Int. Cl.: A61M 25/06, A61B 17/34

(54) **Access device**

(30) Priority: 14.03.2008 US 36900 P; 10.09.2008 US 95886 P; 22.10.2008 US 107632 P
(62) Divisional of application: 09719562.2
(71) Applicant: Access Scientific, Inc., San Diego, CA 92130 (US)
(72) Inventor: Anderson, Janelle, New York, 10024 (US); Bierman, Steven F., Del Mar CA 92104 (US); Huang, Wei, San Diego CA 92122 (US); Pluth, Richard, San Diego CA 92121 (US)
(74) Representative: Müller-Boré & Partner Patentanwälte

(57) **Abstract**

According to the invention, an access device (20) for placing a medical article (40) within a body space is provided, comprising a needle (22) having an elongated needle body (32) with a distal end and a hub (34) from which the needle body extends, the elongated needle body comprising at least one side fenestration (56); a dilator (24) disposed on the needle body, comprising a dilator hub (38) and an elongated dilator shaft (36) that extends from the dilator hub, wherein the dilator shaft and the elongated needle body form one or more spaces, at least one of the spaces communicating with the side fenestration in the needle; and a medical article comprising a tubular section and a hub, the tubular section being disposed on the dilator, wherein at least portions of the dilator and the medical article are configured so as to allow visual determination of the presence of a bodily fluid within the at least one space; and wherein at least one of the needle and dilator further comprise a vent in communication with the at least one space that allows for the escape of air from the space and inhibits the escape of the bodily fluid from the space.

## Description

### CROSS-REFERENCE TO RELATED APPLICATIONS

This application is related to and claims the benefit under 35 U.S.C. § 119(e) to U.S. Provisional Patent Application Serial Nos. 61/036,900 (filed March 14, 2008), 61/095,886 (filed September 10, 2008), and 61/107,632 (filed October 22, 2008), each of which are hereby expressly incorporated by reference in their entireties.

### BACKGROUND

### Field of the Invention

This invention is generally directed to access devices for introducing and/or delivering a medical article (such as, for example, a catheter, cannula, sheath, etc.) into a body space, such as, for example, an artery, vein, vessel, body cavity, or drainage site.

### Description of the Related Art

A preferred non-surgical method for inserting a catheter or vascular sheath into a blood vessel involves the use of the Seldinger or a modified Seldinger technique, which includes an access needle that is inserted into a patient's blood vessel. A guidewire is inserted through the needle and into the vessel. The needle is removed, and a dilator and sheath in combination or separately are then inserted over the guidewire. The dilator and sheath, together or separately, are then inserted a short distance through the tissue into the vessel, after which the dilator and guidewire are removed and discarded. A catheter or other medical article may then be inserted through the sheath into the vessel to a desired location, or the sheath may simply be left in the vessel.

A number of vascular access devices are known. U.S. Patent Nos. 4,241,019, 4,289,450, 4,756,230, 4,978,334, 5,124,544, 5,424,410, 5,312,355, 5,212,052, 5,558,132, 5,885,217, 6,120,460, 6,179,823, 6,210,332, 6,726,659 and 7,025,746 disclose examples of such devices. None of these devices, however, has the ease and safety of use that physicians and other healthcare providers would prefer. Thus, there exists a need for an easier-to-use and safer vascular access device, especially one that would clearly and promptly indicate when a blood vessel has been punctured and one that would reduce accidental needle sticks and other attendant risks of over-wire vascular access.

### SUMMARY

The described embodiments involve several features for an access device useful for the delivery of a catheter or sheath into a space within a patient's body, such as, for example, a blood vessel or drainage site. Without limiting the scope of this invention, its more prominent features will be discussed briefly. After considering this discussion, and particularly after reading the Detailed Description of the Preferred Embodiments section below in combination with this section, one will understand how the features and aspects of these embodiments provide several advantages over prior access devices.

In one embodiment, an access device for placing a medical article within a body space is provided, including a needle, a dilator, and a sheath. The needle can have an elongated needle body with a distal end and a hub from which the needle body extends. The needle body can have an inner surface, an outer surface, and a side hole. The dilator can be disposed on the needle body, and can include a dilator body and a dilator hub. The dilator body can include an inner surface and an outer surface. The sheath can be disposed on the dilator body, and can include a sheath body and a sheath hub. The sheath body can include inner surface and an outer surface. At least one of the surfaces of the needle, dilator, and sheath can be coated at least partially with a surfactant or a lubricious material. Optionally, a space can be defined somewhere between the inner surface of the sheath and the outer surface of the needle, the space being in communication with the side hole.

Further, in these and more specific embodiments, including those discussed above and in the paragraphs which follow, any subcombination of the surfaces can be coated at least partially with a surfactant and/or a lubricious material. For example, the outer surface of the needle and/or the inner surface of the dilator may be at least partially coated with a surfactant and/or lubricious material; and/or the outer surface of the dilator and/or the inner surface of the sheath may be at least partially coated with a surfactant and/or a lubricious material. Accordingly, one, two, three, or all four surfaces may be at least partially coated with a surfactant and/or lubricious material. Furthermore, the inner surface of the needle and/or the outer surface of the sheath may optionally be at least partially coated with a surfactant and/or lubricious material. Generally, as recited herein, a surface of a needle, dilator, or sheath being at least partially coated can include the surface being entirely coated, a majority of the surface being coated, or a minority of the surface being coated. Further, these and similar elements and surfaces in other embodiments described herein can be at least partially coated, as described in relation to the above embodiment.

One aspect of the present invention is an access device for placing a medical article within a body space. The device includes a needle that has an elongated needle body with a distal end and a hub from which the needle body extends. The device further includes a dilator disposed on the needle body. The needle and the dilator are moveable relative to each other from a first position, wherein the distal end of the needle lies distal of the dilator, and a second position, wherein the distal end of the needle lies within the dilator. The dilator includes a dilator hub and an elongated dilator shaft that extends from the dilator hub. The device further includes a locking mechanism that operates between the needle and the dilator to inhibit movement of the needle relative to the dilator when in the second position. The locking mechanism is configured to allow movement of the needle from the first position toward the second position without engagement by the locking mechanism so as to lessen resistance to the movement. The device further includes a sheath disposed on the dilator, the dilator and sheath being moveable relative to each other. Further, at least one of the outer surface of the needle, inner surface of the dilator, outer surface of the dilator, or inner surface of the sheath can be coated at least partially with a surfactant or a lubricious material. The inner surface of the needle and/or the outer surface of the sheath is optionally at least partially coated with a surfactant and/or lubricious material.

Another aspect of the invention is an access device for placing a medical article within a body space. The device includes a needle that has a needle body with a longitudinal axis, a distal tip, and a needle hub from which the needle body extends. The device further includes a dilator that has a dilator shaft and a dilator hub. The dilator shaft is disposed on and slideable along the needle body with the dilator hub being disposed distal of the needle hub. The device further includes a sheath that has a tubular section and a hub. The tubular section is disposed on and slideable along the dilator with the hub being disposed distal of the dilator hub. The device includes a track that extends from the dilator hub in a proximal direction and a locking mechanism operably disposed between the track and the needle hub so as to selectively inhibit proximal movement of the needle relative to the dilator. Further, at least one of the outer surface of the needle, inner surface of the dilator, outer surface of the dilator, or inner surface of the sheath can be coated at least partially with a surfactant or a lubricious material. The inner surface of the needle and/or the outer surface of the sheath is optionally at least partially coated with a surfactant and/or lubricious material.

Yet another aspect of the invention is an access device for placing a medical article within a body space. The device includes a needle that has a distal end and a first fenestration. The device further includes a dilator disposed on and slideable along the needle and has a second fenestration. One of the first and second fenestrations has a greater dimension in at least one direction than the other one of the first and second fenestrations in said direction. The device further includes a sheath being coaxially disposed and longitudinally movable over the dilator. Further, at least one of the outer surface of the needle, inner surface of the dilator, outer surface of the dilator, or inner surface of the sheath can be coated at least partially with a surfactant or a lubricious material. The inner surface of the needle and/or the outer surface of the sheath is optionally at least partially coated with a surfactant and/or lubricious material.

Yet another aspect is an access device for placing a medical article within a body space. The device includes a needle having a distal end and at least one fenestration. The device further includes a dilator that has a shaft disposed on at least a portion of the needle. The device further includes a sheath disposed on at least a portion of the dilator and at least one elongated channel disposed between the needle and an exterior surface of the sheath that extends along at least a substantial portion of the length of the dilator shaft. The channel communicates with the fenestration in the needle and has a span angle of less than 360 degrees about a longitudinal axis of the dilator. Further, at least one of the outer surface of the needle, inner surface of the dilator, outer surface of the dilator, or inner surface of the sheath can be coated at least partially with a surfactant or a lubricious material. The inner surface of the needle and/or the outer surface of the sheath is optionally at least partially coated with a surfactant and/or lubricious material.

Another aspect involves a pre-assembled access device for placing a medical article within a body space. The device includes a needle having a distal end with at least one fenestration and a dilator including a shaft coaxially disposed about at least a portion of the needle. The device further includes a sheath coaxially disposed about at least a portion of the dilator and at least one elongated channel formed between the needle and the exterior surface of the medical article. The channel extends along at least a substantial portion of the length of the dilator shaft. The channel communicates with the fenestration in the needle. The channel is defined at least in part by a groove formed on an inner surface of the medical device, on an outer surface of the dilator, on an inner surface of the dilator, or a combination of such grooves. In some modes, the groove extends only partially around a longitudinal axis of the needle, and in other modes the groove spirals along the axis. Further, at least one of the outer surface of the needle, inner surface of the dilator, outer surface of the dilator, or inner surface of the sheath can be coated at least partially with a surfactant or a lubricious material. The inner surface of the needle and/or the outer surface of the sheath is optionally at least partially coated with a surfactant and/or lubricious material.

A further aspect involves an access device for placing a medical article within a body space. The access device comprises a needle having a distal end and a longitudinal axis, and a dilator disposed on at least a portion of the needle and having an outer surface. A sheath is disposed on at least a portion of the dilator and has an inner surface. At least a portion of the inner surface of the medical article or a portion of the outer surface of the dilator has a dissimilar shape to that of an adjacent portion of the outer surface of the dilator or inner surface of the sheath (respectively) so as to form a gap therebetween, which extends along the longitudinal axis. Further, at least one of the outer surface of the needle, inner surface of the dilator, outer surface of the dilator, or inner surface of the sheath can be coated at least partially with a surfactant or a lubricious material. The inner surface of the needle and/or the outer surface of the sheath is optionally at least partially coated with a surfactant and/or lubricious material.

A releasable interlock can be provided in some embodiments to inhibit relative rotational movement between the needle and the dilator, at least when the needle is inserted into a patient. By inhibiting such relative rotational movement, communicating fenestrations in the needle and the dilator can be held in alignment to provide a simplified channel through which the blood or fluid may flow. Thus, when the needle enters a blood vessel or drainage site in the patient, blood or other body fluid quickly flows into the channel. The resulting blood or fluid flash is visible through the sheath (or catheter) to indicate that the needle tip has entered the vessel or drainage site.

For example, but without limitation, the dilator can comprise, in some embodiments, a dilator hub and dilator having one or more side fenestrations. The dilator hub may have a luer connection and a releasable locking mechanism. The releasable locking mechanism can be configured to releasably engage and secure the dilator to another part, such as the needle hub. When the needle hub and the dilator hub are releasably locked to prevent rotation therebetween, at least a portion of one or more of the side fenestrations in the dilator are aligned with at least a portion of one or more side fenestrations in the needle. The locking mechanism can also be configured to inhibit unintentional relative axial movement between the needle and the dilator.

The sheath preferably, but not necessarily, includes a sheath hub. The sheath may be made partially or completely from a clear, translucent, semi-opaque, or transparent material. Such transparent, translucent, semi-opaque and clear materials allow a clinician the ability to see when blood or other body fluids flows into the needle, through the needle fenestration(s), through the side dilator fenestration(s), and into the viewing space between the dilator and sheath. The sheath may also have radiopaque stripes so disposed as not to obscure the viewing space. Further, the sheath may have a silicone coat.

Further, in some embodiments of the present invention an access device can be provided for placing a medical article within a body space. The access device can include a needle, a dilator, and a medical article. The needle can have an elongated needle body with a distal end, as well as a hub from which the needle body extends. The elongated needle body further can have at least one side fenestration. The dilator can be disposed on the needle body and include both a dilator hub and an elongated dilator shaft that extends from the dilator hub. The dilator shaft and the elongated needle body can then together form one or more spaces, and at least one of these spaces can communicate with the side fenestration in the needle. The medical article can include a tubular section and a hub. The tubular section of the medical article can be disposed on the dilator. Further, at least a portion of the dilator and medical article can be configured to allow an observer to visually determine the presence of a bodily fluid within the space. Additionally, at least one of the needle or dilator can include a vent in communication with the space. The vent allows for the escape of air from the space, and can inhibit the escape of the bodily fluid from the space. Further, these embodiments can include surfactants and silicone coats, as described herein.

These and other aspects of the present invention will become readily apparent to those skilled in the art from the following detailed description of the preferred embodiments, which refers to the attached figures. The invention is not limited, however, to the particular embodiments that are disclosed.

### BRIEF DESCRIPTION OF THE DRAWINGS

These and other features, aspects, and advantages of the access device disclosed herein are described below with reference to the drawings of preferred embodiments, which are intended to illustrate and not to limit the invention. Additionally, from figure to figure, the same reference numerals have been used to designate the same components of an illustrated embodiment. Like components between the illustrated embodiments are similarly noted as the same reference numbers with a letter suffix to indicate another embodiment. The following is a brief description of each of the drawings.

FIGURE 1A is a perspective view of a preferred embodiment of an access device configured in accordance with the present invention and shows a pre-loaded guidewire section coaxially aligned with a needle, a dilator, and a medical article.

FIGURE 1B is a plan view of the embodiment depicted in FIGURE 1A.

FIGURE 2A is a plan view of the needle from FIGURE 1A and shows a fenestration near a distal end.

FIGURE 2B is a side view of the needle from FIGURE 1A and shows a fin near a proximal end.

FIGURE 2C is a cross-sectional view taken along the lines 2C-2C in FIGURE 2A.

FIGURE 2D is an enlarged plan view of a portion of the needle of FIGURE 2A and shows the fenestration.

FIGURE 2E is an enlarged plan view of the needle hub of the needle of FIGURE 2A.

FIGURE 2F is an enlarged side view of the needle hub of the needle of FIGURE 2A.

FIGURE 2G is an enlarged proximal end view of the needle hub of the needle of FIGURE 2A.

FIGURE 3A is a plan view of the dilator from FIGURE 1A and shows a fenestration near a distal end. FIGURE 3A also shows longitudinally arranged grooves in the luer surface for venting air from between the dilator and sheath.

FIGURE 3B is a cross-sectional view taken along the lines 3B-3B in FIGURE 3A.

FIGURE 3C is an enlarged plan view of a portion of the dilator from FIGURE 3A and shows the fenestration and longitudinal channel.

FIGURE 3D is an enlarged end view of the dilator hub from FIGURE 3A.

FIGURE 3E is a perspective view of another embodiment of the dilator hub that includes a locking spin nut configured to secure to a sheath that has a corresponding screw thread.

FIGURE 3F is a cross-sectional view taken along the lines 3F-3F in FIGURE 3A and shows the grooves equally spaced about the circumference of the luer surface.

FIGURE 4A is a plan view of the sheath from FIGURE 1A and shows a sheath hub connected to a proximal end of a sheath.

FIGURE 4B is a cross-sectional view taken along the lines 4B-4B in FIGURE 4A.

FIGURE 4C is an enlarged end view of the sheath from FIGURE 4A.

FIGURE 4D is an enlarged perspective view of a proximal portion of the sheath from FIGURE 4A.

FIGURE 5A is a perspective view of the guidewire section from FIGURE 1A and shows a guidewire hub connected to a proximal end of a guidewire.

FIGURE 5B is a plan view of the guidewire section of the embodiment depicted in FIGURE 5A.

FIGURE 6A is a perspective view of a track from FIGURE 1A.

FIGURE 6B is a plan view of the track in FIGURE 6A and shows a locking mechanism for locking the needle relative to the dilator.

FIGURE 6C is a side view of the track in FIGURE 6B.

FIGURE 6D an enlarged view of the locking mechanism from FIGURE 6B.

FIGURE 6E is an enlarged view of another locking mechanism that locks the guidewire section in a pre-loaded state.

FIGURE 7A is a plan view of the access device from FIGURE 1A and shows the locking mechanism from FIGURE 6E with the guidewire section locked to the track in the pre-loaded state.

FIGURE 7B is a side view of the access device and locking mechanism from FIGURE 7A.

FIGURE 7C is a cross-sectional view through the access device of FIGURE 7A and shows the guidewire hub disposed between an element and stop of the track.

FIGURE 7D is an enlarged end view of the access device from FIGURE 7B and shows two arms extending from the track and around at least a portion of the guidewire hub.

FIGURE 8A is a plan view of the embodiment depicted in FIGURE 1A illustrating the insertion of the distal end of the access device into a patient.

FIGURE 8B is an enlarged view of the embodiment depicted in FIGURE 8A focusing on the area of the access device adjacent to the patient.

FIGURE 8C is an enlarged view of a portion of the embodiment depicted in FIGURE 8B and illustrates the needle opening or fenestration aligned with the dilator opening or fenestration in hidden lines.

FIGURE 8D is an enlarged cross-sectional view of a portion of the embodiment depicted in FIGURE 8C and shows the needle opening or fenestration aligned with the dilator opening or fenestration so as to allow fluid to flow from inside the needle to a channel formed between the sheath and dilator.

FIGURE 8E is a graph showing the rate fluid is drawn up a channel with a gap height width of 0.002 inches.

FIGURE 8F is a graph showing the rate fluid is drawn up a channel with a gap height width of 0.001 inches.

FIGURE 8G is a graph showing the rate fluid is drawn up a channel with a gap height width of 0.0005 inches.

FIGURE 8H is an enlarged cross-sectional view of a portion of the embodiment depicted in FIGURE 8C taken through a region distal of the channel in the dilator.

FIGURE 8I is an enlarged view of the embodiment depicted in FIGURE 8A focusing on the area where the needle hub is locked to the dilator hub when the needle hub is in the first position.

FIGURE 8J is a cross-sectional view of the embodiment depicted in FIGURE 8I.

FIGURE 9A is a side view of the embodiment depicted in FIGURE 1A illustrating the guidewire advanced from the needle tip in a distal direction.

FIGURE 9B is an enlarged view of the embodiment depicted in FIGURE 9A focusing on the area where the guidewire hub is locked to the needle hub when the needle hub is in the first position.

FIGURE 9C is a cross-sectional view of the embodiment depicted in FIGURE 9B.

FIGURE 10A is a side view of the embodiment depicted in FIGURE 1A illustrating the dilator and sheath being advanced distally relative to the needle body from the position illustrated in FIGURE 9A.

FIGURE 10B is an enlarged rear view of the embodiment depicted in FIGURE 10A focusing on the area where the needle hub is locked to the track when the needle hub is in the second position.

FIGURE 11A is a side view of the embodiment depicted in FIGURE 1A illustrating the removal of the guidewire, needle body, and dilator from the sheath.

FIGURE 11B is an enlarged view of the portion of the embodiment illustrated in FIGURE 11A showing the needle tip covered by the dilator during removal of the guidewire, needle body, and dilator from the sheath.

FIGURE 12A is an enlarged plan view that illustrates another embodiment of the aligned openings or fenestrations in the needle and dilator.

FIGURE 12B is an enlarged cross-sectional view along lines 13B-13B in FIGURE 12A and shows the needle opening or fenestration aligned with the dilator opening or fenestration so as to allow fluid to flow from inside the needle to a channel formed between the sheath and dilator.

FIGURE 13A is an enlarged plan view that illustrates another embodiment of the aligned openings or fenestrations in the needle and dilator.

FIGURE 13B is an enlarged cross-sectional view along lines 13B-13B in FIGURE 13A and shows the needle opening or fenestration aligned with the dilator opening or fenestration so as to allow fluid to flow from inside the needle to a channel formed between the sheath and dilator

FIGURE 14A is an enlarged plan view that illustrates another embodiment of the channel formed between the dilator and the sheath.

FIGURE 14B is a cross-sectional view along lines 14B-14B in FIGURE 14A and shows the thickness of the channel extending into the sheath.

FIGURE 15A is an enlarged plan view that illustrates another embodiment of the channel formed between the dilator and the sheath.

FIGURE 15B is a cross-sectional view along lines 15B-15B in FIGURE 15A and shows the thickness of the channel extending into both the dilator and the sheath.

FIGURE 16A is an enlarged plan view that illustrates another embodiment of the channel formed between the dilator and the sheath.

FIGURE 16B is a cross-sectional view along lines 16B-16B in FIGURE 15A and shows a plurality of equally spaced channels in the form of splines extending into the dilator.

FIGURE 17 is an enlarged cross-sectional view through another embodiment of the access device and shows the channel formed between a sheath and a dilator that have dissimilar shapes.

FIGURE 18A is an enlarged plan view of a portion of another embodiment of the access device and illustrates another embodiment of a channel this time formed between the needle and the dilator.

FIGURE 18B is an enlarged cross-sectional view through the embodiment of FIGURE 18A taken at 18B-18B.

FIGURE 18C is an enlarged cross-sectional view through the embodiment of FIGURE 18A taken at 18C-18C.

FIGURE 18D is an enlarged perspective view of a needle hub configured to form part of the needle depicted in FIGURE 18A.

FIGURE 18E is a plan view of the dilator of FIGURE 18A.

### DETAILED DESCRIPTION OF THE PREFERRED EMBODIMENTS

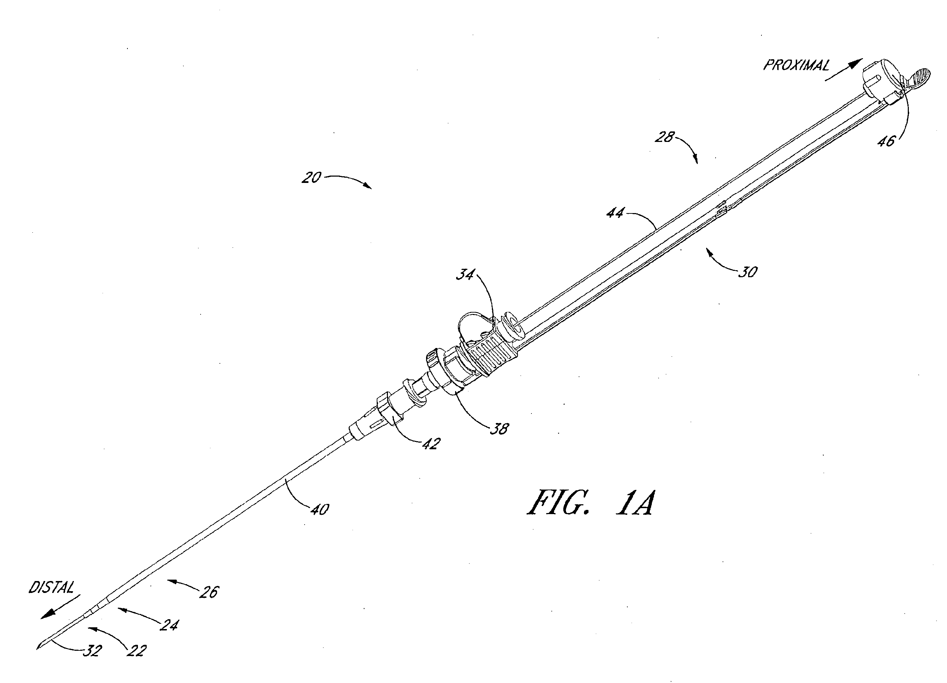

The present disclosure provides an access device for the delivery of a medical article (e.g., catheter or sheath) to a blood vessel or drainage site. FIGURE 1A illustrates an access device 20 that is configured to be inserted into a blood vessel (e.g., a vein or an artery) in accordance with a preferred embodiment of the present invention. While the access device is described below in this context (i.e., for vascular access), the access device also can be used to access and place a medical article (e.g., catheter or sheath) into other locations within a patient's body (e.g., a drainage site) and for other purposes (e.g., for draining an abscess).

The present embodiment of the access device is disclosed in the context of placing an exemplary single-piece, tubular medical article into a body space within a patient. Once placed, the tubular article can then be used to receive other medical articles (e.g., catheters, guidewires, etc.) to provide access into the body space and/or be used to provide a passage way for introducing fluids into the body space or removing (e.g., draining) fluids from the body space. In the illustrated embodiment, the tubular medical article is a sheath or catheter that is configured primarily to provide a fluid passage into a vein. The principles of the present invention, however, are not limited to the placement of single piece sheaths or catheters, or to the subsequent insertion of a medical article via the sheath or catheter. Instead, it will be understood by one of skill in this art, in light of the present disclosure, that the access device disclosed herein also can be successfully utilized in connection with placing one or more other types of medical articles, including other types of sheaths, fluid drainage and delivery tubes, and single or multi-lumen catheters directly in the patient or indirectly via another medical article.

For example, but without limitation, the access device disclosed herein can also be configured to directly or indirectly place central venous catheters, peripherally inserted central catheters, hemodialysis catheters, surgical drainage tubes, tear-away sheaths, multi-piece sheaths, scopes, as well as electrical conduit for wires or cables connected to external or implanted electronic devices or sensors. As explained above, the medical articles listed above may be directly placed in the patient via the dilator, needle, and guidewire of the access device or subsequently placed within the patient via a medical article that was placed within the patient via the dilator, needle, and guidewire of the access device.

Further, the embodiments disclosed herein are not limited to co-axial insertion of a single medical article. For example, two catheters may be inserted in the patient via an inserted sheath or a second catheter may be inserted in the patient via an inserted first catheter. Further, in addition to providing a conduit into the vessel or other body space, the medical article inserted via the dilator, needle, and guidewire can form a lumen that is in addition to the lumen(s) of the subsequently inserted medical article. One skilled in the art can also find additional applications for the devices and systems disclosed herein. Thus, the illustration and description of the access device in connection with a sheath (e.g., for micro puncture applications) is merely exemplary of one possible application of the access device.

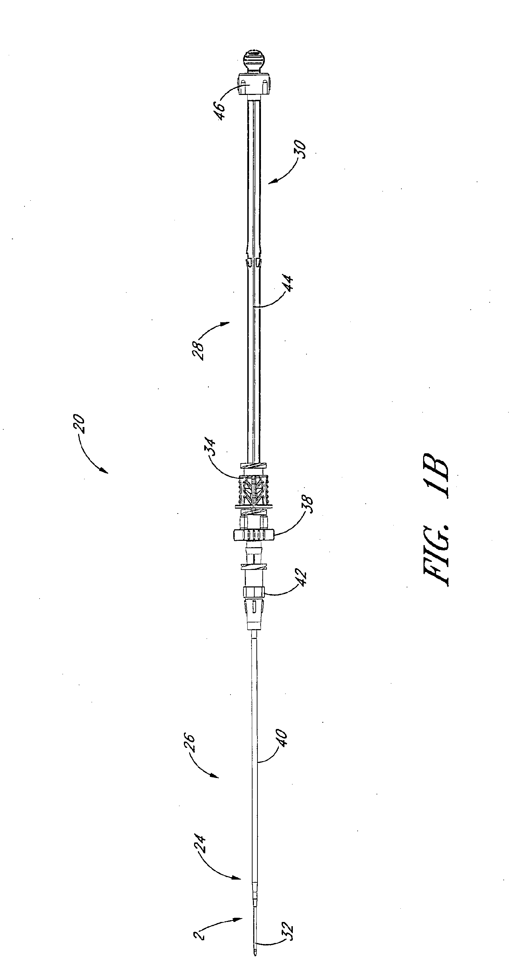

FIGURES 1A and 1B illustrated a preferred embodiment of an access device 20. The access device 20 comprises a needle 22, a dilator 24, and a sheath 26. In the illustrated embodiment, the access device also includes a guidewire section 28 and a track 30. As best seen in FIGURE 1B, the dilator 24 is preferably coaxially mounted on the needle 22, and the sheath 26 is coaxially mounted on the dilator 24. The telescoping nature of the access device's components can also be accomplished by arranging the components with their axes arranged substantially parallel rather than coaxially (e.g., a monorail-type design).

Each of these components includes a luminal fitting at a terminal end or transition (i.e., a hub) and elongated structure that extends from the fitting. Thus, in the illustrated embodiment, the needle 22 includes a needle body 32 that extends distally from the needle hub 34, the dilator 24 includes a dilator shaft 36 that extends distally from a dilator hub 38, and the sheath 26 includes a sheath body 40 that extends distally from a sheath hub 42. The guidewire section 28 comprises a guidewire 44 and preferably a guidewire hub or cap 46. In the illustrated embodiment, the guidewire hub 46 is disposed on the proximal end of the guidewire 44; however, in other applications, the hub 46 can be disposed at a location between the ends of the guidewire 44.

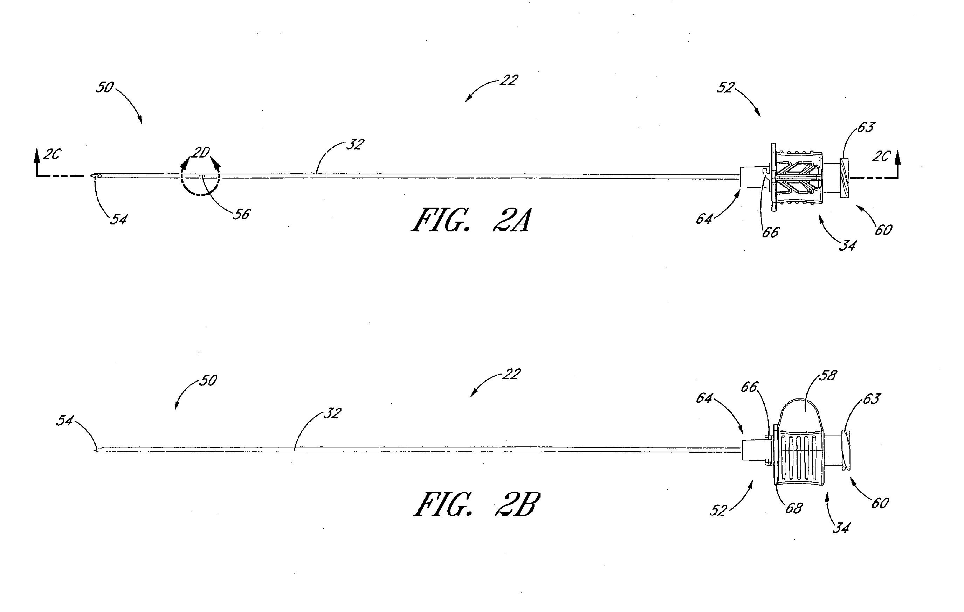

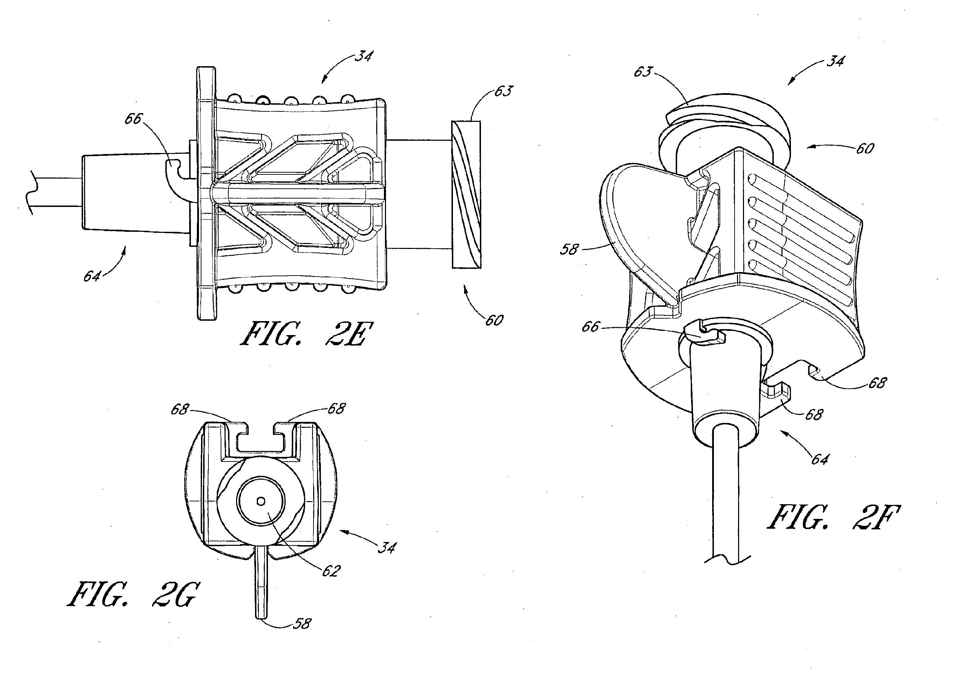

FIGURES 2A-2G illustrate the needle body 32 and needle hub 34 of the needle 22, which are configured in accordance with a preferred embodiment of the access device, in isolation from the other components of the access device 20. As best seen in FIGURES 2A and 2B, the needle hub 34 is disposed on a proximal end of the needle body 32. The needle body 32 terminates at a distal end near a distal portion 50 of the needle 22, and the needle hub 34 lies at a proximal portion 52 of the needle 22.

The needle body 32 preferably has an elongated tubular shape having a circular, constant-diameter inner bore and a circular, constant-diameter exterior surface. In other embodiments, however, the needle body 32 can have other bore and exterior shapes (such as, for example, but without limitation, an oval cross-sectional shape). The interior or exterior of the needle can also include grooves or channels. The grooves or channels may guide fluids within the needle bore either around or to certain structures of the needle 22 or within the needle 22 (e.g., around the guidewire). In some embodiments, the grooves or channels may assist in maintaining a desired orientation of the needle 22 with respect to the dilator.

The needle body 32 has a sufficiently long length to access a targeted subcutaneous body space and has a sufficient gauge size to withstand the insertion forces when accessing the body space without causing undue trauma. For many applications, the needle body can have a length between 3- 20 cm, and more preferably between 3-10 cm. For example, to access a body space (e.g., a vessel) in the thorax of an adult human, the needle body 32 preferably has a length of 7 cm or greater, and more preferably has a length of 9 cm or greater, and most preferably has a length of 9 to 10 cm. The size of the needle preferably is 18 gauge or smaller, and more preferably between 18-28 gauge, and most preferably between 18-26 gauge for micro-puncture applications (peripheral IVs). For applications with a neonate, the length and gauge of the needle body 32 should be significantly shorter and smaller, for example preferably between 3-4 cm and between 26-28 gauge.

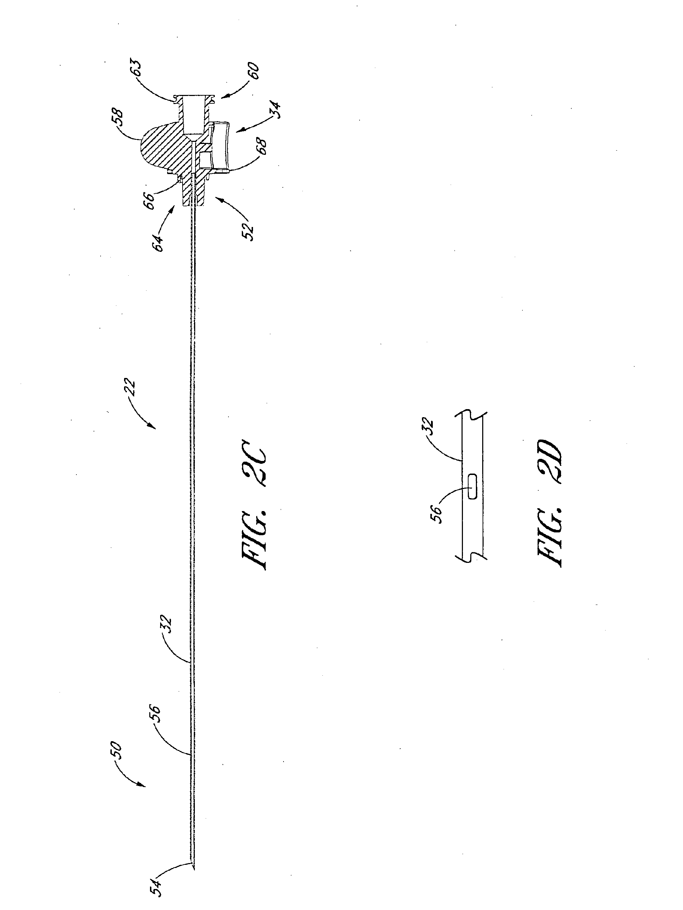

As best seen in FIGURES 2A and 2D, the needle body 32 includes at least one fenestration or opening 56 near a distal end of the needle body 32. The fenestration 56 extends through the wall- of the needle body 32 and can have a variety of shapes and orientations on the needle body 32, as described in detail below. In addition, the needle body 32 can have a bevel tip 54 disposed on the distal portion 50.

As is illustrated in FIGURES 2A and 2B, a fin 58 is preferably disposed at a circumferential location around the needle hub 34 that is aligned with the circumferential locations of the bevel on the needle tip and the opening or fenestration 56 in the needle. That is, the fin 58 is indexed with the bevel and fenestration. During use, the physician or healthcare provider can determine the orientation of the beveled needle tip (and the fenestration 56) by noting the orientation of the exposed fin 58 even though the bevel is inside the vessel and the fenestration is covered by the sheath and/or dilator. For example, in the illustrated embodiment, an orientation of the fin 58 away from the patient coincides with a bevel up orientation of the needle tip within the vessel. The fenestration 56 is also on the same side as the fin 58, as seen in FIGURE 2C.

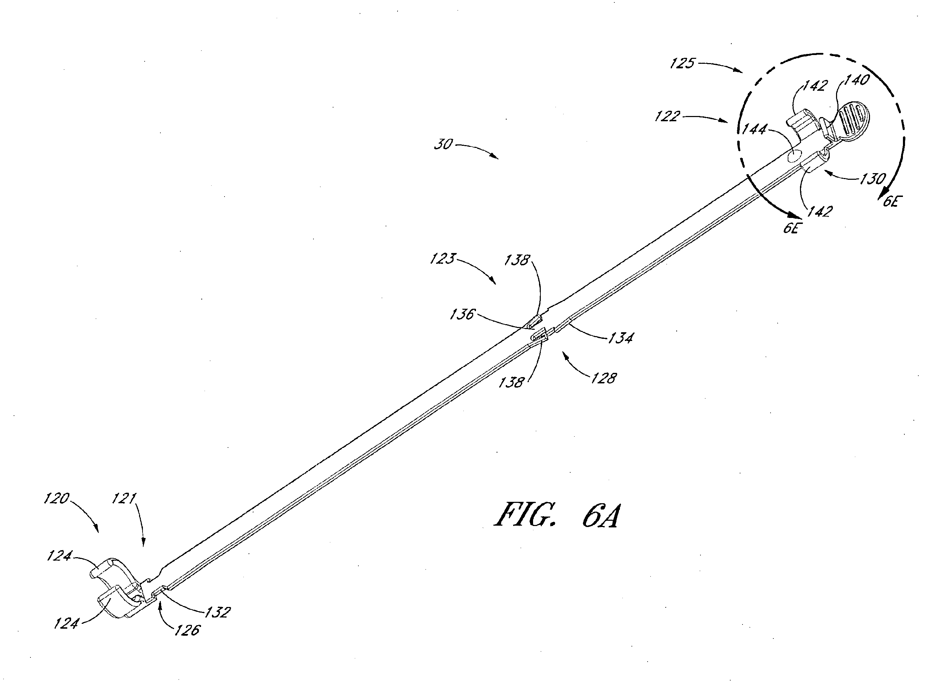

The fin 58 also provides a grasping region to manipulate the needle hub 34. For example, a physician or healthcare provider can place an index finger and thumb on the sides of the fin 58 to stabilize the needle hub 34, relative to the dilator 24 and/or sheath 26. In the illustrated embodiment, as the dilator/sheath slides distally over the needle, the needle hub 34 slides relatively along the track 30 between a first position 121 and a second position 123 (example portions illustrated in FIGURE 6A). The fin 58 can be held when performing the insertion step (which will be described below). In addition, the fin 58 can be used to stabilize the needle hub 34 while rotating the dilator hub 38. Furthermore, the fin 58 can be used by a physician or healthcare provider as an aid to grasp the access device 20 when the needle hub 34 is disposed at any position along the track 30.

FIGURE 2D is an enlarged view of the side opening or fenestration 56 in the needle body 32. The one or more fenestration 56 provides a path through the side of the needle body 32. The fenestration 56 illustrated in FIGURE 2D has an oblong shape. The shape of the side opening 56, however, is not limited to the illustrated embodiment and may be round, oblong, square, or another shape.

With specific reference now to FIGURES 2E-2G, the needle hub 34 preferably includes locking structures at the proximal portion and distal portion of the needle hub 34. These locking structures may be a luer-thread-type or another type of connections.

The locking structure on the proximal portion 52 of the needle hub 34 allows the physician or healthcare provider to secure another medical article to the proximal end of the needle hub 34. For example, the needle hub 34 in the illustrated embodiment includes an annular flange or lip 63. The lip 63 is threaded to allow the needle hub 34 to attach to other medical articles with a corresponding luer-nut locking feature. Additionally, a physician or healthcare provider may attach a syringe or monitoring equipment to the locking structure on the proximal end to perform other procedures as desired. The needle hub 34 can also include a septum at its proximal end and/or a side port if these features are desirably for a particular application.

The locking structure on the distal portion of the needle hub 34 allows the physician or healthcare provider, for example, to lock the needle hub 34 to the dilator hub 38 when the needle hub 34 is in the first position 121. In the illustrated embodiment, the locking structure includes a latch element 66 on the needle hub 34. The latch element 66 releasably locks the needle hub 34 to the dilator hub 38. The locking structure allows the healthcare provider to advance the needle into a patient while grasping the needle hub 34, the dilator hub 38 or both.

As explained below in greater detail, the guidewire 44 is introduced through a hollow portion 62 of the needle hub 34, through the needle body 32, and into a punctured vessel. The guidewire 44 allows the healthcare provider to guide the dilator 24 and sheath 26 into the vessel.

The needle hub 34 may also comprise two tangs 68 that allow the needle hub 34 to slide along the track 30 between a first position 121 and a second position 123. While in the preferred embodiment the two tangs 68 of the needle hub 34 are engaged with the track 30 between the first position 121 and the second position 123, in other embodiments the needle hub 34 is only engaged with the track 30 over a portion of the length of the track 30 between the first position 121 and the second position 123. The sliding interconnection between the track 30 and the needle hub 34 also can be accomplished using other cooperating structures (e.g., a corresponding pin and tail of dovetail connection).

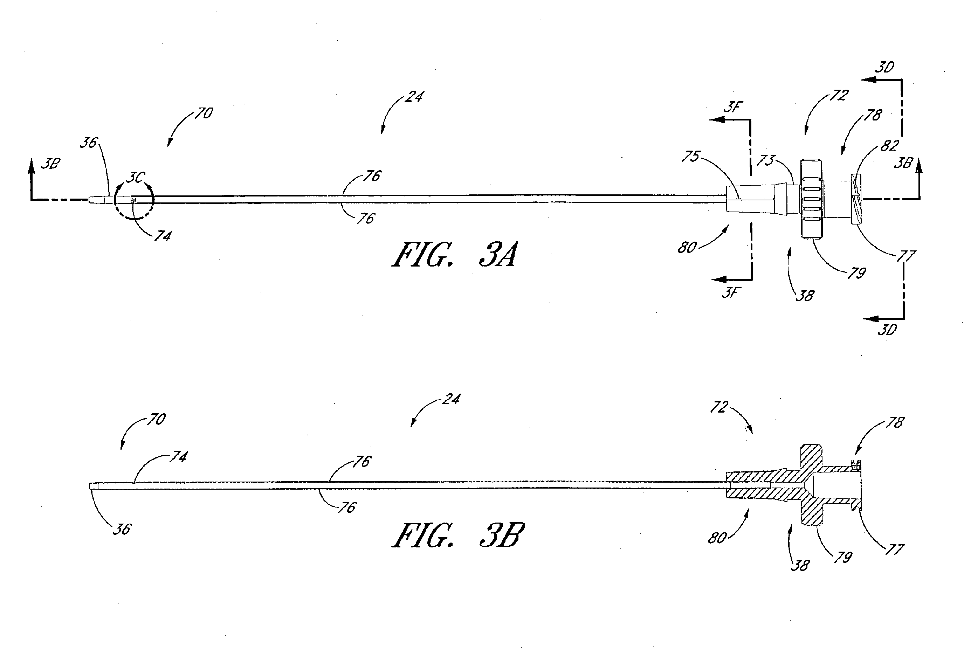

FIGURE 3A is a plan view of the dilator 24 of the embodiment depicted in FIGURE 1A. FIGURE 3B is a cross-sectional view of the dilator 24 of the embodiment depicted in FIGURE 3A, taken along line 3B-3B. As shown in FIGURES 3A and 3B, the illustrated dilator 24 comprises a dilator shaft 36, a dilator hub 38, a distal region 70, and a proximal region 72. In the illustrated embodiment, the dilator shaft 36 includes a side openings or fenestrations 74; however, in other embodiments, the dilator shaft 36 can include fewer or greater numbers of fenestrations 74. For example, the dilator shaft 36 may not include a fenestration 74 where a blood flash chamber(s) is disposed within the dilator (as will be described in more detail below).

The dilator hub 38 may comprise one or more vents. In the illustrated embodiments, the vents in the dilator hub 38 are formed by grooves 75. Additionally, the dilator shaft 36 may comprise one or more longitudinal channels formed in the outer surface of the dilator shaft 36. In the illustrated embodiment, the channel is an open channel. The side walls of the open channel are formed by ridges 76. In the illustrated embodiment, the ridges 76 define generally smooth, arcuate exterior surfaces that interface with the sheath 26; however, in other embodiments, the ridges can have other shapes (e.g., can define more pronounced apexes). Once assembled within a sheath body 40, the open channel in the dilator shaft 36 is closed by the inside diameter of the sheath body 40.

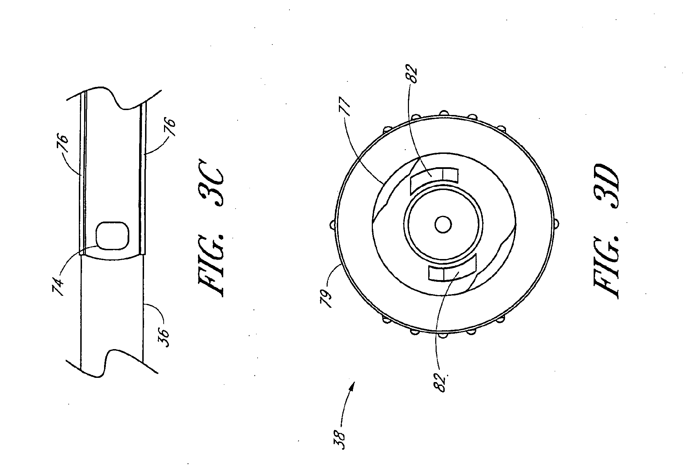

FIGURE 3C is an enlarged plan view of a portion of the embodiment illustrated in FIGURE 3A. As noted above, the illustrated dilator shaft 36 comprises one or more side openings 74 and one or more channels formed between ridges 76. The side opening or fenestration 74 provides a fluid path through the side of the dilator shaft 36. The shape of the side opening 74 is not limited to the illustrated embodiment and may be round, oblong, square, or have another shape. The opening or fenestration 74 illustrated in FIGURE 3C has an oblong shape.

In the illustrated embodiment, the opening 74 in the dilator shaft 36 has an oblong shape with its major axis being non-parallel relative to the major axis of the oblong opening 56 in the needle 22. For example the needle opening 56 may extend in a longitudinal direction and the dilator opening 74 may extend in a circumferential direction or vice versa. In other words, the long axis of the dilator opening 74 is disposed generally perpendicular to the long axis of the needle opening 56. As explained in connection with additional embodiments below, these openings 56, 76 can have other shapes, sizes and orientations that preferably obtain a significant degree of overlap to account for manufacturing tolerances and rotational misalignments. For this reason, it is preferred that one of the fenestrations has a greater dimension in at least one direction than the other one of the fenestrations in the same direction. Accordingly, in the illustrated embodiment, the needle fenestration 56 has a longer longitudinal dimension than the longitudinal dimension of the dilator fenestration 74.

The channel formed between the ridges 76 extends in a proximal direction from a point distal to the opening 74. The ridges 76 in the illustrated embodiment are disposed along the dilator shaft 36 and on opposite sides of the dilator shaft 36 so as to balance the dilator shaft 36 within the sheath. In the illustrated embodiment, the ridges 76 form two channels there between. Balancing the dilator within the sheath allows the dilator to apply equal pressure to the inside circumference of the sheath.

The dilator hub 38 may include locking structures at the proximal region 72 and the distal region of the dilator 24. Each locking structure may be a luer type or other type of connection. In the illustrated embodiment, the dilator hub 38 comprises a first luer connection 78, a second luer connection 80, a lip 77, and a base 79. The first luer connection 78 engages to the needle hub 34 on the needle 22 illustrated in FIGURE 2E. The second luer connection 80 is disposed distal to the first luer connection 78. In some embodiments, the second luer connection 80 (e.g., a male luer slip connector) can be configured to engage to the sheath hub 42 (e.g., a female luer slip connector) on the sheath 26 illustrated in FIGURE 1A. Additionally, the male-female lure slip connectors on these components can be reversed.

FIGURE 3D is an enlarged proximal end view of the dilator 24 of FIGURE 3A. As shown most clearly in FIGURE 3D, the dilator hub 38 comprises an opening 82 that releasably engages the latch element 66 on the needle hub 34 illustrated in FIGURE 2E-2F to secure the dilator hub 38 to the needle hub 34 when the needle hub 34 is in the first position 121. Again, the male-female lure slip connectors on the dilator hub and the needle hub 34 can also be reversed in other embodiments.

The color of the dilator 24 may be selected to enhance the contrast between the blood or other fluid and the dilator 24. During blood flash, for example, blood is observed flowing between the dilator 24 and the sheath to confirm proper placement of the needle in a blood vessel. To increase the visibility of the fluid as the fluid flows between the sheath and dilator 24, the sheath is preferably manufactured from a clear or transparent material with the dilator 24 having a color that contrasts with the color of the fluid. For example, the dilator 24 may have a white color to enhance its contrast with red blood. Other colors of dilator 24 could be employed depending on the color of the fluid and the degree of contrast desired. Further, only a portion of the dilator in the region of the blood flash can have the contrasting color with the remainder having a different color. For embodiments that have a channel formed between the needle and dilator 24, the dilator 24 may be manufactured of a clear or transparent material similar to the sheath to allow the physician to observe the blood flash through both the sheath and dilator 24.

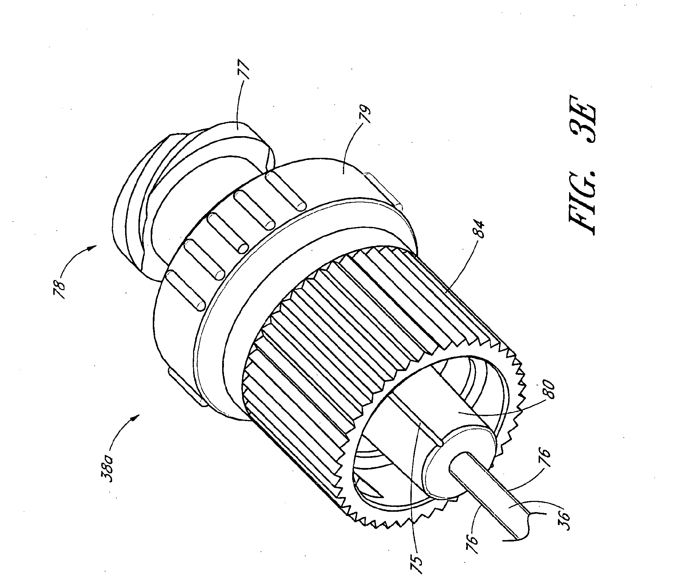

FIGURE 3E is an enlarged perspective view of another embodiment of a dilator hub 38A. The dilator hub 38A is similar to the dilator hub 38 illustrated in FIGURE 3A except that the dilator hub 38A further includes a spin nut or collar 84. The proximal end of the spin nut 84 rotates about an annular groove 73 in the dilator hub 38 (see FIGURE 3A). Once disposed within the annular groove 73, the spin nut 84 is inhibited from moving in the distal direction but is free to rotate about the dilator hub 38A. The spin nut 84 can have an interengaging element that locks to a corresponding interengaging element on the sheath 26. In the illustrated embodiment, the spin nut 84 includes an internal thread which engages with an external thread on the sheath hub 42 on the sheath 26 illustrated in FIGURE 1A.

The dilator 24 or sheath 26 may separately, or together, form one or more passages to allow air or gas to escape or vent from between the dilator 24 and sheath 26 and/or between the needle and the dilator. The one or more passages may further be sized to inhibit the flow of a liquid, such as blood, while allowing air to pass therethrough. The one or more passages may be in the wall of the sheath 26, the sheath hub, the dilator hub 38, an exposed section of the dilator shaft, and/or formed between adjacent surfaces of the dilator 24 and sheath 26. For example, FIGURE 3A shows longitudinally arranged grooves 75 that are formed between adjacent surfaces of the dilator 24 and sheath 26. Such venting passages can also be labyrinth. The adjacent surfaces form a luer slip connection between the sheath 26 and dilator 24.

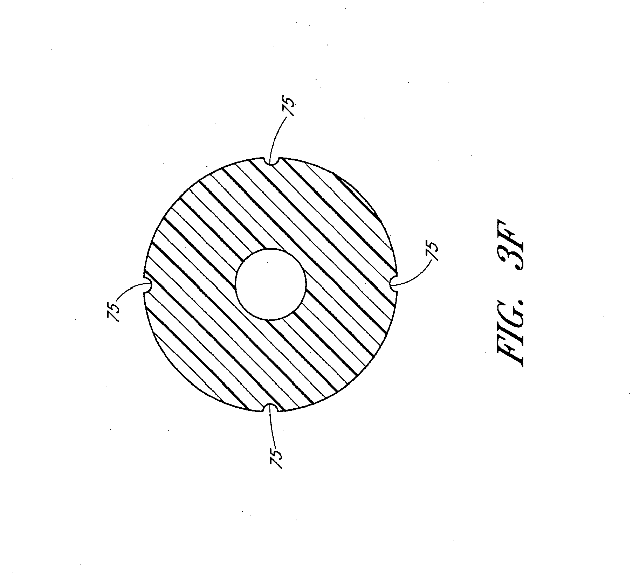

FIGURE 3F is a cross-sectional view taken along lines 3F-3F in FIGURE 3A and shows the grooves 75 equally spaced, though not required to be equally spaced, about the circumference of the luer slip surface. The grooves 75 are sized to allow air to escape from between the dilator and the medical article, such as a sheath, when the blood flash occurs. As mentioned above, the one or more passages need not be in the form of a surface groove 75 and instead may be in the form of an opening or passageway.

In the illustrated embodiment, the one or more passages allow air to pass through the luer connection between the sheath and dilator hubs. In the illustrated embodiment, a distal end of the passage 75 is located on the distal side of the luer connection with the proximal end of the passage 75 being located on the proximal side of the luer connection.

The one or more passages may be sized to filter blood or other liquid or may include a filter or other structure that inhibits the passage of a liquid while allowing the passage of air.. For example, the sheath itself may include one or more passages in the form of small openings, pores or porous material. Depending on the size of the one or more passages and the expected size of the fluid molecules and formed elements (e.g. red blood cells), the one or more small opening, pores or porous material in the sheath can form a porous vent that allows air to pass yet retain blood.

A method of manufacturing a ridged dilator will now be described. First, an extrusion process is used to create a long tubular body having one or more longitudinal grooves or channels on its outer diameter (OD) or within the substance of the dilator. The long tubular body exceeds the required length of a single dilator and preferably has a length that is many times greater than the length of a single dilator. A manufacturing die is employed in the extrusion process having geometry that reflects the desired geometry for the inside and outside diameters of the dilator and the thickness and circumferential span of the longitudinal grooves or channels or interior channels. In the illustrated embodiment of FIGURES 1-11, the long tubular body includes two longitudinal OD channels on opposite sides of the body to enhance the balance of the dilator within the sheath. However, a single channel can provide a visible indicator for the blood flash. The two channels preferably extend along the length of the extruded tubular body. While the illustrated embodiment includes one or more channel disposed between the dilator and the sheath, one or more channels can in addition or in the alternative be formed between the needle and the dilator, within the dilator, and/or within the sheath. In some embodiments, the dilator 24 thus is made partially or completely from clear, translucent, transparent, or semi-opaque material to visualize the fluid flash within the channel.

With reference back to the illustrated embodiment, the extruded tubular body is cut to the appropriate length for a single dilator. In the preferred method, the two OD grooves extend for the entire length of the cut dilator.

A tipping process is then employed on an end of the cut dilator to reform the tip. An end of the cut dilator is forced into a die/mandrel having geometry that matches the desired geometry of the tip of the finished dilator. The desired geometry is selected depending on, for example, the inside diameter of the sheath. It is desirable for the sheath and dilator to form a close fit or seal near the tip to promote blood flow in the proximal direction up the channel formed between the grooved dilator and sheath. Preferably, the OD of the dilator in the tip region tapers in the distal direction.

When in the die/mandrel, thermal energy is applied to the tip to reform the tip to match the die/mandrel. The thermal energy may be applied by any known technique, including using radiant heating from an infrared or RF heat source. As part of the tipping process, the dilator in the tip region is reformed so that the grooves are essentially removed. With the grooves removed, the dilator is able to form the close fit or seal with the sheath near the tip. The grooves are maintained along the remainder of the dilator on the proximal side of the location where the tip of the sheath 26 sits on the dilator. After removal from the die/mandrel, the tip end of the dilator may be cleaned and cut as necessary to remove any manufacturing remnants.

The one or more fenestrations in the dilator is cut through the dilator near the tip region and in or near the groove. Each fenestration may be cut by any known means, including a drill or laser. Further, the cutting device may be moved with respect to the dilator or vice versa to achieve an oblong or other shape for the fenestration.

The end of the dilator opposite from the tip end can be flared to facilitate over molding the dilator hub onto the dilator.

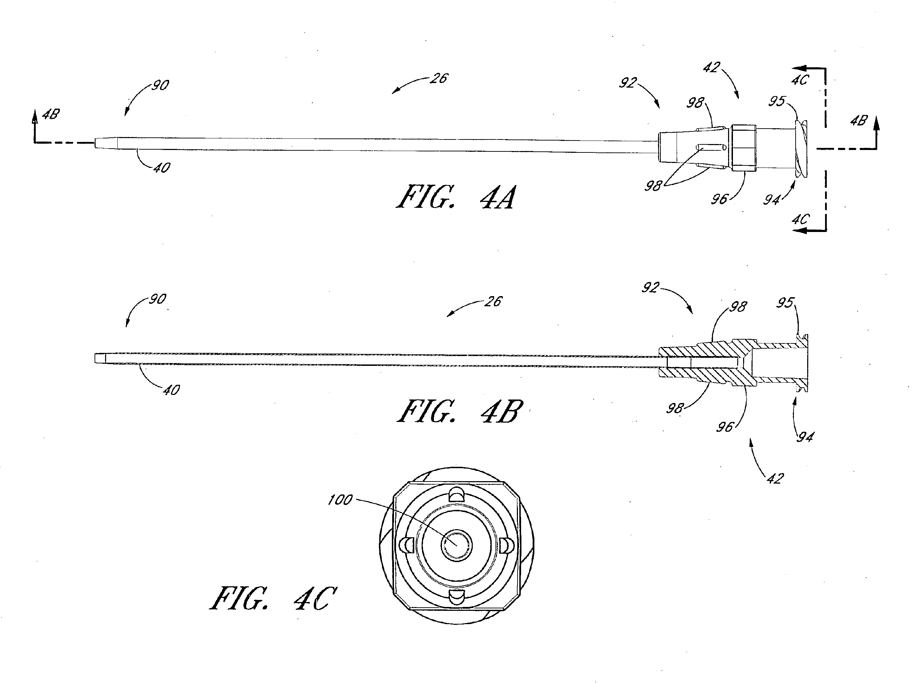

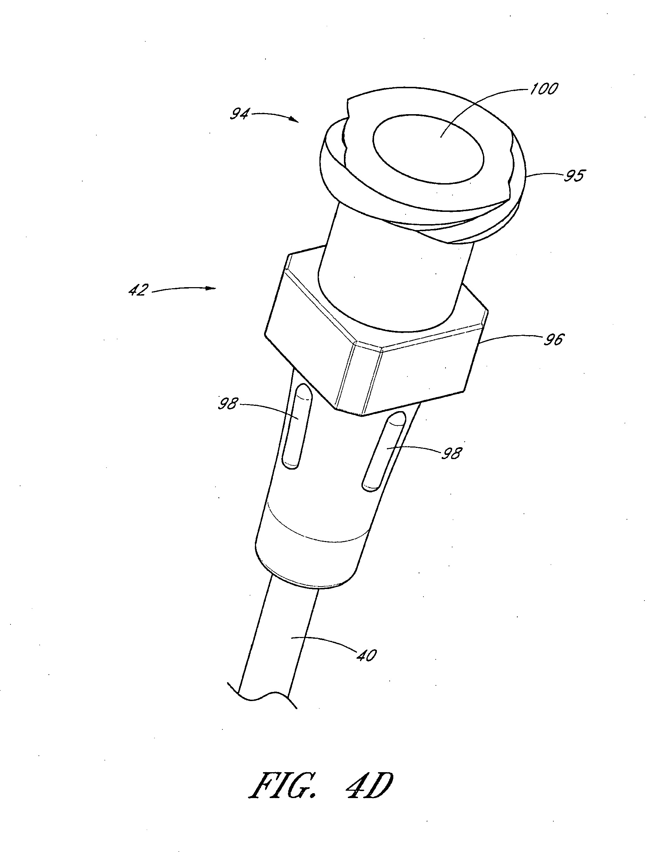

FIGURE 4A is a plan view of the sheath 26 of the embodiment depicted in FIGURE 1A. FIGURE 4B is a cross-sectional view of the sheath 26 of the embodiment depicted in FIGURE 4A, taken along line 4B-4B. FIGURE 4C is an enlarged proximal end view of the sheath 26 of FIGURE 4A. Figure 4D is an enlarged perspective view of the sheath hub 42 of the sheath 26 of FIGURE 4A. As shown in FIGURES 4A-4D, the sheath 26 may comprise a sheath body 40, a sheath hub 42, a distal portion 90, and a proximal region 92. The sheath body 40 may be made partially or completely from clear, translucent, transparent, or semi-opaque material. The sheath body 40 can also include one or more radiopaque markers, such as, for example, barium sulfate stripes. In a preferred embodiment, the sheath includes two such radiopaque stripes disposed on diametrically opposite sides of the body 40.

The sheath body 40 may be a single piece sheath through which a catheter or other medical article (e.g., a guidewire) is inserted into the vessel. In such an embodiment, the sheath body 40 forms a conduit for insertion of the catheter or other medical article (e.g., a guidewire). In addition to providing a conduit, the sheath or a portion of the sheath can form a lumen that is in addition to the lumen(s) of the catheter. For example, an equivalent to a triple lumen catheter can be formed by inserting a dual lumen catheter through the sheath body 40 with the sheath body 40 itself forming a third lumen.

It may be advantageous to remove a portion or the entire sheath body 40 depending on the type of catheter or medical article that is to be inserted into the vessel after employing the access device 20. For example, after the catheter or other medical article is inserted into the vessel, a portion of the sheath body 40 can be separated or peeled-away and removed. A peel-away sheath can include perforations, serrations, skives, or other structures, or include other materials (e.g., PTFE with bismuth) to allow the physician or healthcare provider to remove easily a portion or the entire sheath body 40.

The sheath hub 42 may include a luer slip connection and a lock member 94. The locking member 94 may comprise a locking or attaching structure that mates or engages with a corresponding structure. For example, the lock member 94 can be a luer connection 94 which can be configured to engage with the second luer connection 80 of the dilator hub 38.

The sheath hub 42, as best seen in FIGURE 4C and 4D, preferably is designed so that the locking mechanism or second luer connection 80 of the dilator hub 38 can enter the sheath hub 42 substantially unobstructed. However, in use, once the sheath hub 53 is placed at a desired location over the dilator shaft 36, the physician or healthcare provider can push, pull, or twist the sheath hub 42 and possibly disengage or engage the locking member 94 with a corresponding connector on another medical article. The locking member 94 can be, for example, a luer connection, a protruding bump, dent, etc., that creates a mechanical fit so that the dilator hub 38 and the sheath hub 42 are releasably interlocked. In the illustrated embodiment, the locking member 94 of the sheath hub 42 comprises a luer connection. The sheath hub 42 preferably engages with the corresponding second luer connection 80 on the dilator hub 38. Preferably, the locked position can be disengaged or engaged by pulling, squeezing, pushing or twisting the dilator hub 38 relative to the sheath hub 42.

In some embodiments, the sheath hub 42 can comprise a lip 95. The lip 95 can be threaded to allow the sheath hub 42 to attach to other medical articles with a corresponding locking feature.

The sheath hub 42 preferably comprises one or more surface features to allow the physician or healthcare provider to easily grasp or manipulate the sheath 26 and/or access device 20. In the illustrated embodiment, the sheath hub 42 includes a squared grip 96 and ridges 98.

In additional embodiments, the sheath hub 42 may comprise radially extending wings or handle structures to allow for easy release and removal of the sheath body 40 from other parts of the access device 20. In some applications, the wings are sized to provide the healthcare provider with leverage for breaking apart the sheath hub 42. For example, the sheath hub 42 may comprise a thin membrane connecting the halves of the sheath hub 42. The membrane is sized to keep the halves of the sheath hub 42 together until the healthcare provider decides to remove the sheath hub 42 from the access device. The healthcare provider manipulates the wings to break the membrane and separate the sheath hub 42 into removable halves.

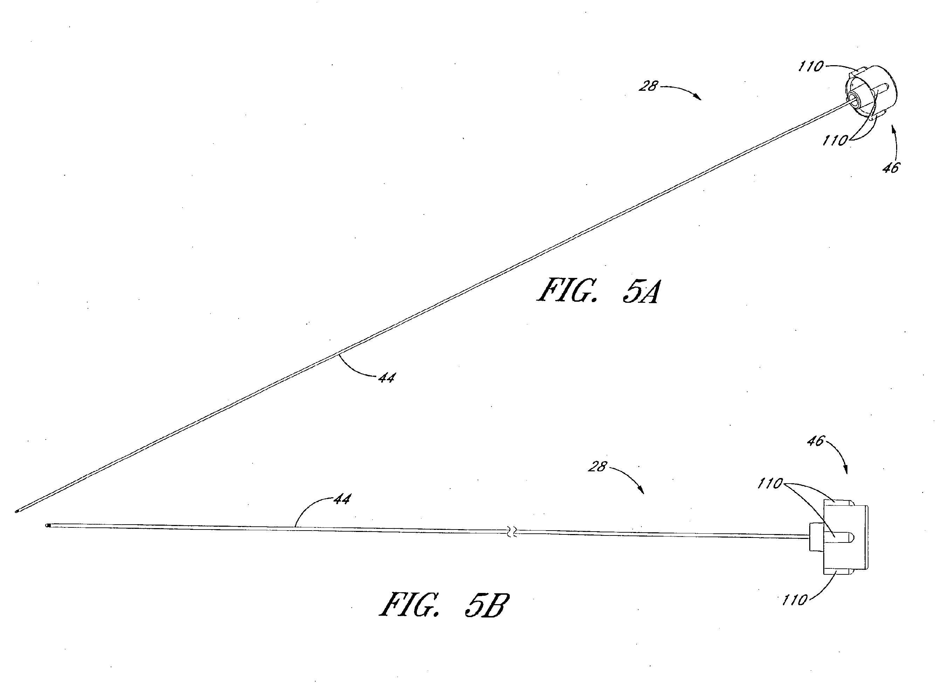

FIGURE 5A is a perspective view of the guidewire section 28 of the embodiment depicted in FIGURE 1A. FIGURE 5B is a plan view of the guidewire section 28 depicted in FIGURE 5A, which preferably includes the guidewire hub 46. The guidewire hub 46 can comprise one or more surface features to allow the physician or healthcare provider to easily grasp or manipulate the guidewire hub 46 and/or access device 20. In the illustrated embodiment, the guidewire hub 46 comprises one or more ridges 110. In a pre-loaded state, the outer surface of the guidewire hub 46 engages with a locking mechanism 130 on the track 30 when the guidewire hub 46 is in a third position 125 (example third position illustrated in FIGURE 6A).

In some embodiments, the guidewire 44 may form a close fit with the inside diameter of the needle body so as to provide a self-aspirating function when retracted. For example, an outside diameter of the guidewire 44 may be selected to form a close fit with the needle along the length of the guide wire or along only a portion of the guidewire 44.

In some embodiments, the distal end portion of the guidewire can have a reduced diameter in comparison to other sections of the guidewire. The size of such reduced diameter section can be selected to permit fluid to pass to the fenestration 56 in the needle body even when the guidewire has been advanced beyond the distal tip of the needle.

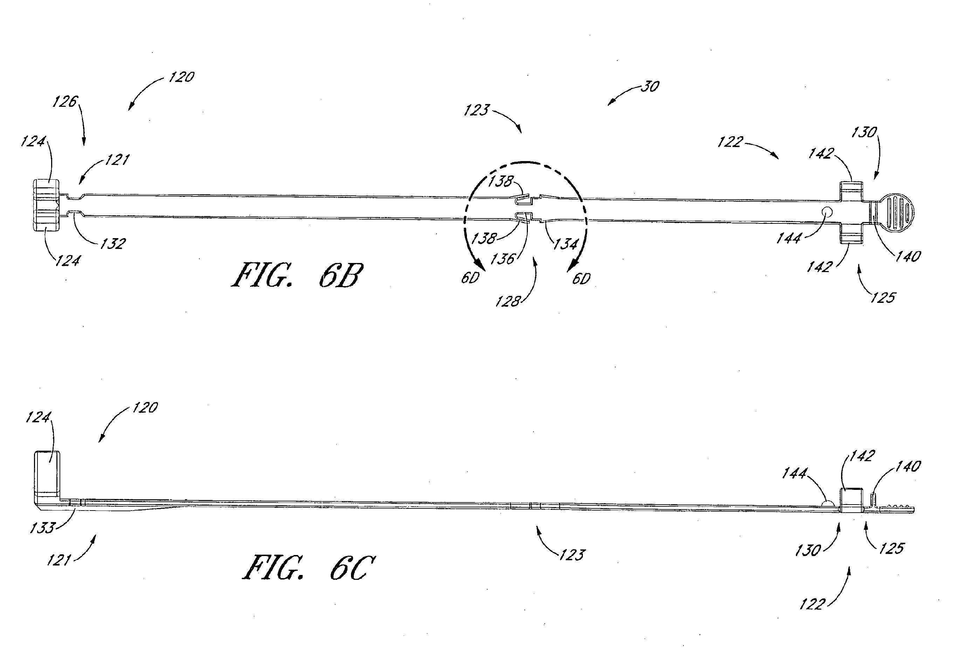

FIGURE 6A is a perspective view of the track 30 of the embodiment depicted in FIGURE 1A. Figure 6B is a plan view of the track 30 illustrated in FIGURE 6A. FIGURE 6C is a side view of the track 30 illustrated in FIGURE 6A. As shown in FIGURES 6A-6C, the track 30 in the illustrated embodiment comprises a distal portion 120, a proximal portion 122, a distal locking member 124 that connects the track to the dilator hub 38, a locking mechanism 128 that inhibits further proximal and distal movement of the needle hub 34 once the needle hub 34 is slid from the first position 121 to the second position 123 along the track 30, and a locking mechanism 130 that allows the guidewire hub 46 to attach to the track 30 when the guidewire hub is in the pre-loaded state or third position 125. Preferably, the track is made of polycarbonate material; however, as explained below, other materials can be used.

The track 30 may further include a track section 132 of reduced width as shown most clearly in FIGURES 6A and 6B. The reduced width facilitates assembly of the needle hub to the track 30. The illustrated embodiment includes a rib 133 on the distal portion 120 of the track 30. The rib 133 provides additional structural reinforcement between the distal locking member 124 and the remainder of the track 30.

As illustrated in FIGURE 1A, the distal locking member 124 connects to the dilator 24 and allows the track 30 to extend proximally from the dilator 24. For example, the locking member 124 can comprise two curved arms 124 that connect to the dilator hub 38 between the dilator hub lip 77 and the dilator hub base 79. The locking member 124 limits movement of the track 30 in a distal or proximal direction relative to the dilator hub 38 but allows the track 30 to rotate freely around the dilator hub 38.

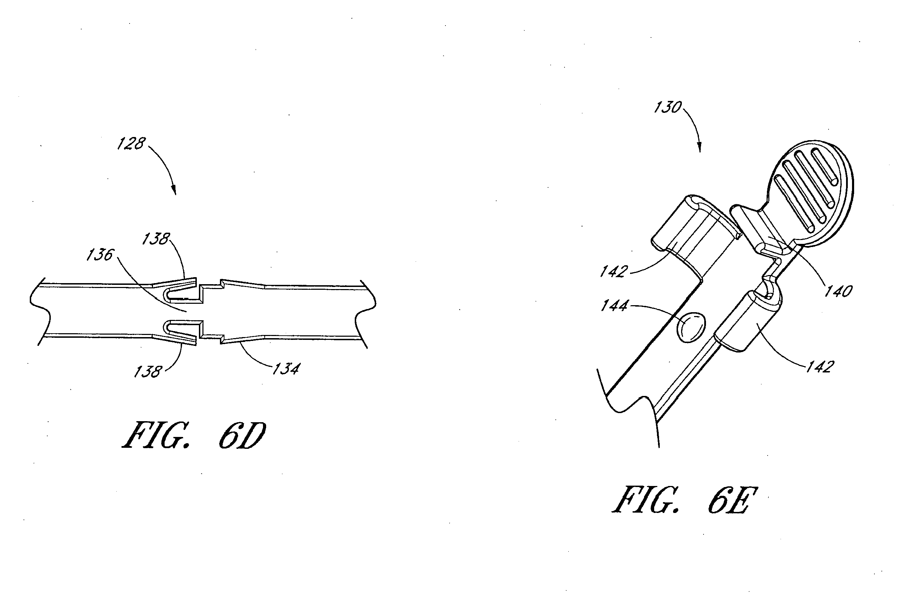

FIGURE 6D is an enlarged view of a portion of the embodiment depicted in FIGURE 6B. As shown, the locking mechanism 128 is formed by varying the width of the track in the region of the second position 123. For example, the illustrated embodiment includes a track section 134 of increasing width in the distal direction, a track section 136 of reduced width distal to the track section 134 of increasing width, and two finger elements 138. The two finger elements 138 project from the distal end of the track section 136 toward the proximal end of the track 30 and flare away from the longitudinal axis of the track 30.

FIGURE 6E is an enlarged view of a portion of the enbodiment depicted in FIGURE 6B. The locking mechanism 130 is formed by a clip, clasp or other structure that engages with a portion of the guidewire hub or with a portion of the track 30 when the guidewire hub is in the third position. Some or all of the engagement structure may be part of the track 30, be part of the guidewire hub, or be split between the track 30 and guidewire hub. In the illustrated embodiment, the locking mechanism 130 extends from the track 30 and engages with the guidewire hub. The locking mechanism 130 comprises a rectangular element 140 protruding from the track 30, two track arms 142 projecting from the track 30 distal to the rectangular element 140, and a stop 144 protruding from the track 30 distal to the track arms 142.

In the illustrated embodiment, the locking mechanism between the needle hub and the dilator resides on the proximal side of the dilator hub. In other embodiments, however, the locking mechanism can be disposed at other locations as well. For example, where the locking mechanism includes two pivotal levers which are joined by a locking hinge, the locking mechanism can be disposed radially relative to the needle hub. In such an embodiment, one lever is pivotally coupled to the dilator and the other lever is pivotally coupled to the needle. When the needle hub is moved away from the dilator hub, the levers straighten to a point where the hinge locks. A similar effect can be obtained by a tether limiting proximal movement of the needle hub relative to the dilator beyond a particular point, thereby locking the components together. In a further embodiment, an elongated structure can extend parallel to the needle body from the needle hub within the dilator. Once the needle hub is moved a sufficient distance away from the dilator, additional structure of the locking mechanism (e.g., a detent) engages the elongated structure to inhibit further movement of the needle relative to the dilator. Accordingly, as illustrated by these additional embodiments, the locking mechanism operating between the needle and the dilator can be disposed at a variety of locations relative to the dilator hub.

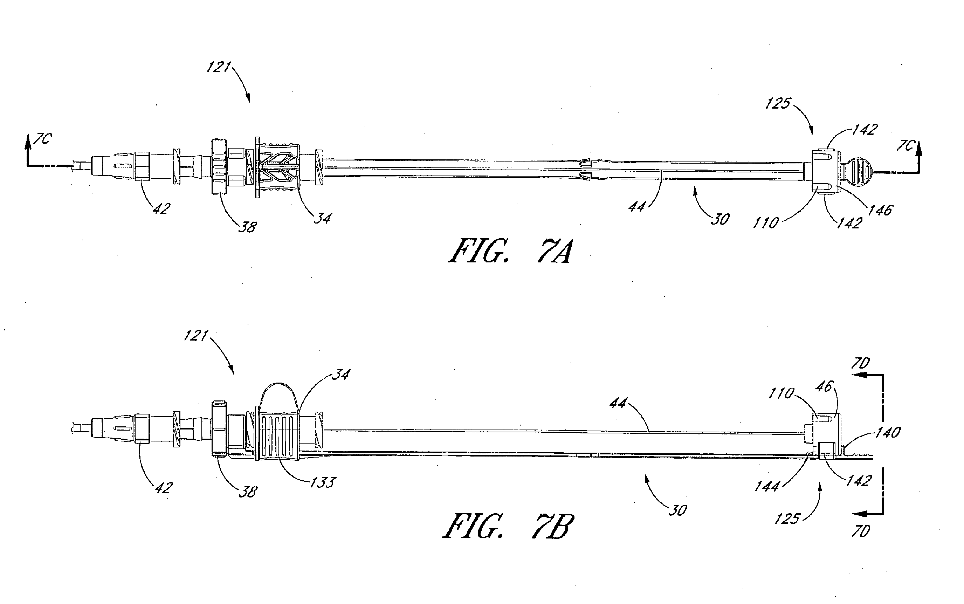

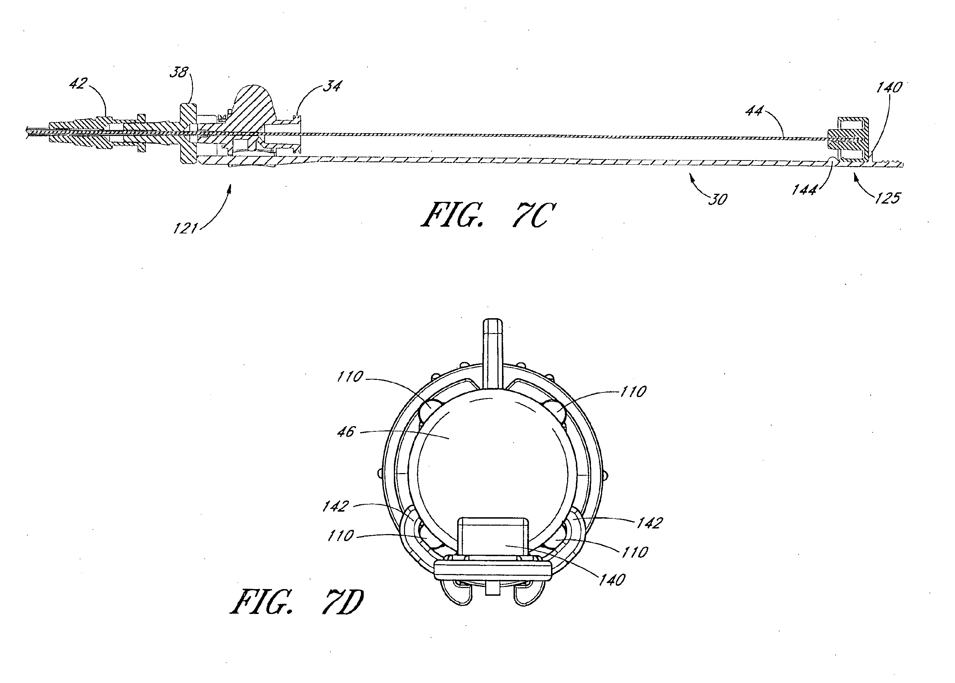

FIGURE 7A is an enlarged plan view of the access device of the embodiment depicted in FIGURE 1A pre-loaded with the guidewire. FIGURE 7B is a side view of the embodiment depicted in FIGURE 7A. FIGURE 7C is a cross-sectional view of the embodiment depicted in FIGURE 7A along line 7C-7C. FIGURE 7D is a proximal end view of the access device 20 of FIGURE 7A. In this pre-loaded state, the guidewire hub 46 is locked to the track 30 when the guidewire hub 46 is located in a third position 125. In this position, the guidewire hub 46 can be secured to the track 30 between the rectangular element 140 and the stop 144. For example, the guidwire hub 46 can releasably lock between the rectangular element 140 and the stop 144. In addition, the track arms 142 can further secure the guidewire hub 46 to the track 30. This locking mechanism can arrest unintended rotational and axial movement of the guidewire 44 at least in the distal direction when the guidewire hub 46 is in the third position 125. Of course, the healthcare provider may disengage the guidewire hub 46 from the track 30 to allow distal movement of the guidewire through the access device 20.

In the preloaded-state illustrated in FIGURES 7A-7C, the needle hub 34 is locked to the dilator hub 38 when the needle hub 34 is in the first position 121. Preferably, in the locked position, the openings or fenestrations in the needle and dilator are in register or in alignment with each other. When locked, the needle 22 and the dilator 24 are inhibited from at least unintentional rotational and axial movement relative to each other. By preventing unintentional rotation of the dilator hub with respect to the needle 34, the fenestrations or openings maintain their general alignment.

In the pre-loaded state, the dilator hub 38 is secured to the sheath hub 42. This can inhibit at least unintentional rotational and axial movement between the dilator 24 and the sheath 26. In embodiments where the sheath hub 42 and the dilator 24 have only a luer slip connection, the dilator 24 and sheath hub 42 may rotate relative to each other.

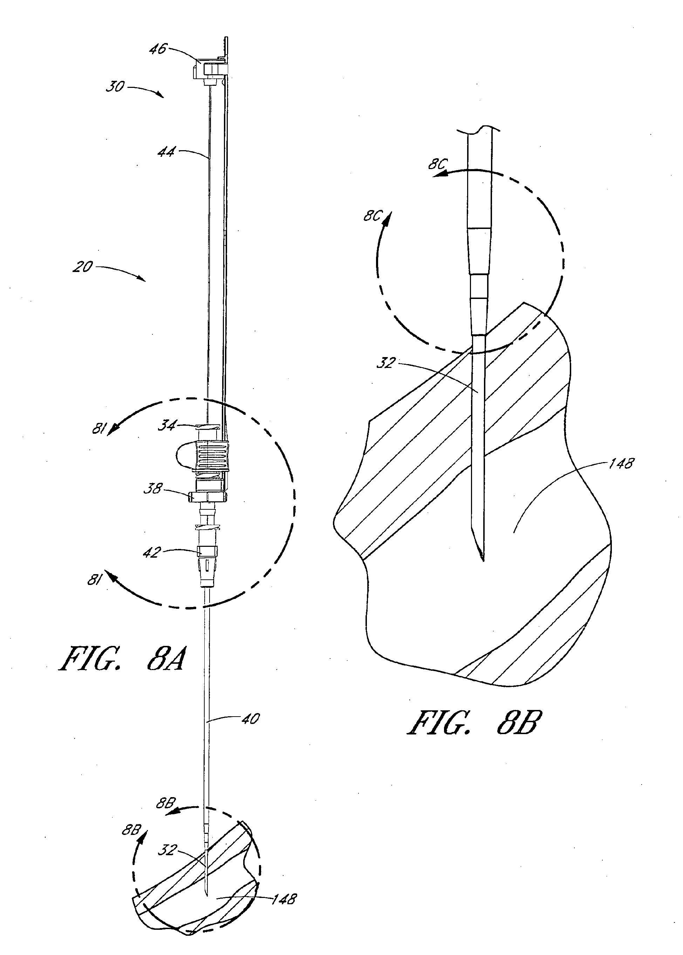

FIGURE 8A is a plan view of the embodiment depicted in FIGURE 1A that illustrates an operational step of one method of using the access device 20. FIGURE 8A depicts the needle body 32 of the access device 20 inserted into a vessel 148, such as a vein. While the described method refers to vascular access, the access device 20 also can be used to access and place a catheter or sheath into other locations within a patient's body (e.g., for draining an abscess) and for other purposes.

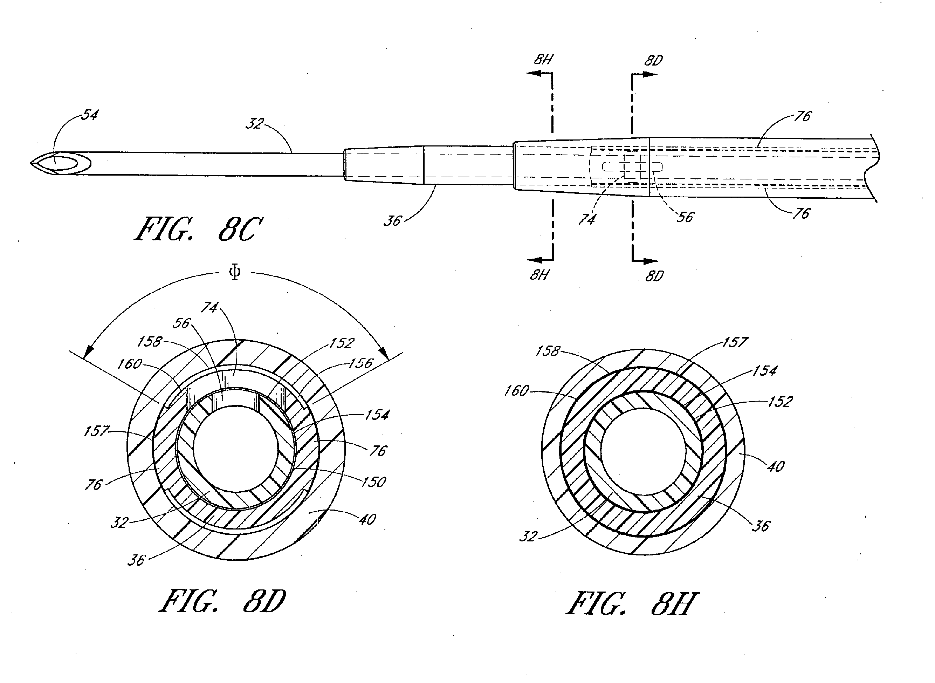

FIGURE 8B is an enlarged plan view of the portion of the embodiment illustrated in FIGURE 8A which is circled by line 8B-8B. FIGURE 8C is an enlarged plan view of the portion of the embodiment illustrated in FIGURE 8B which is circled by line 8C-8C. FIGURE 8D is an enlarged cross-sectional view of the embodiment depicted in FIGURE 8C along line 8D-8D.

As noted above, the needle body 32 comprises one or more side openings 56 in its side wall. The dilator shaft 36 comprises one or more side openings 74. The side openings 56, 74 may have the same or different shapes as well as aspect ratios. In the illustrated embodiment, the side opening 56 in the needle body 32 has a different aspect ratio than the side opening 74 in the dilator shaft 36. The side opening 56 in the needle body 32 is elongated in one direction (e.g., substantially parallel to the longitudinal axis of the needle body 32). The side opening 74 in the dilator shaft 36 is elongated in a different direction (e.g., along the circumference of the dilator shaft 36). Having offset elongated openings 56, 74 in the needle body 32 and the dilator shaft 36 increases the likelihood that the openings 56, 74 in the needle body 32 and dilator shaft 36 will be sufficiently aligned so that blood flows through the needle side opening 56 and dilator side opening 74. FIGURES 8A-D illustrate the alignment between only one set of corresponding side openings. Other sets of side openings can also be aligned or be misaligned depending upon the relative orientations of the needle body 32 and the dilator shaft 36.

In the illustrated embodiment, the dilator shaft 36 is coaxially positioned to minimize an annular space 150 between the needle body 32 and the dilator shaft 36. The inner surface 152 of the dilator shaft 36 need not, though it can, lie directly against the outer-surface 154 of the needle body 32. Preferably, in this embodiment, the annular space 150 between the outer-surface 154 of the needle body 32 and the inner surface 152 of the dilator shaft 36 is minimized to inhibit the flow of blood or its constituents (or other bodily fluids) into the annular space 150 between the dilator shaft 36 and needle body 32. Advantageously, this feature minimizes the blood's exposure to multiple external surfaces and reduces the risk of contamination, infection, and clotting.

As illustrated in FIGURE 8A, the dilator shaft 36 is coaxially mounted to the needle body 32 such that at least part of one side opening 56 disposed on the needle body 32 is rotationally aligned with at least part of one side opening 74 on the dilator shaft 36. Preferably, the needle body 32 and dilator shaft 36 maintain rotational alignment so that blood flows through the needle side opening 56 and dilator side opening 74.

The sheath body 40, as noted previously, is preferably made partially or completely from clear, semi-opaque, translucent, or transparent material so that when blood flows into the needle body 32, (1) through the needle side opening 56, (2) through the dilator side opening 74, and (3) into a channel 156, the physician or healthcare provider can see the blood. In some modes, the channel 156 is formed between the dilator shaft 36 and the sheath body 40 and defined by one or more ridges 76 on the dilator shaft 36. In some modes, the channel 156 is formed within a wall of the dilator shaft 36 with the dilator shaft 36 preferably comprising a transparent material. Blood will indicate to the physician or healthcare provider that the bevel tip 54 of the needle body 32 has punctured a vessel 148.

In some embodiments, the needle body 32 and dilator shaft 36 may (both) have multiple side openings where some or all of these side openings can be rotationally aligned.

The channel 156 can have an axial length that is almost coextensive with the length of the sheath 26. In other embodiments, the channel 156 can be significantly smaller than the elongated channel 156 just described. For example, but without limitation, the channel 156 can be disposed within a distal, mid and/or proximal portion(s) of the sheath 26. The channel 156 alternatively can have a linear, curved or spiral shape along an axial length of the sheath 26 or can be formed by a plurality of such shapes. The channel 156 may have various thicknesses and span angles. The thickness of the channel 156 can range from almost close to zero to 0.010 inches. Preferably, the channel 156 has a thickness of about 0.0005 to about 0.003 inches. More preferably, the channel 156 can have a thickness of about 0.001 inches to about 0.002 inches. The channel 156 can have a span angle φ about the axis of the dilator 24 of about 30 degrees to about 210 degrees or more, but preferably less than 360 degrees. More preferably, the channel 156 can have a span angle φ of about 60 to 150. In the illustrated embodiment, the channel 156 spans 120 degrees. The thickness and span angle φ can be chosen so as to optimize the capillary action that occurs within the channel 156 as fluid (e.g., whole blood) enters the channel 156 as may further be selected based on the expected pressure in the body cavity and viscosity of the liquid.

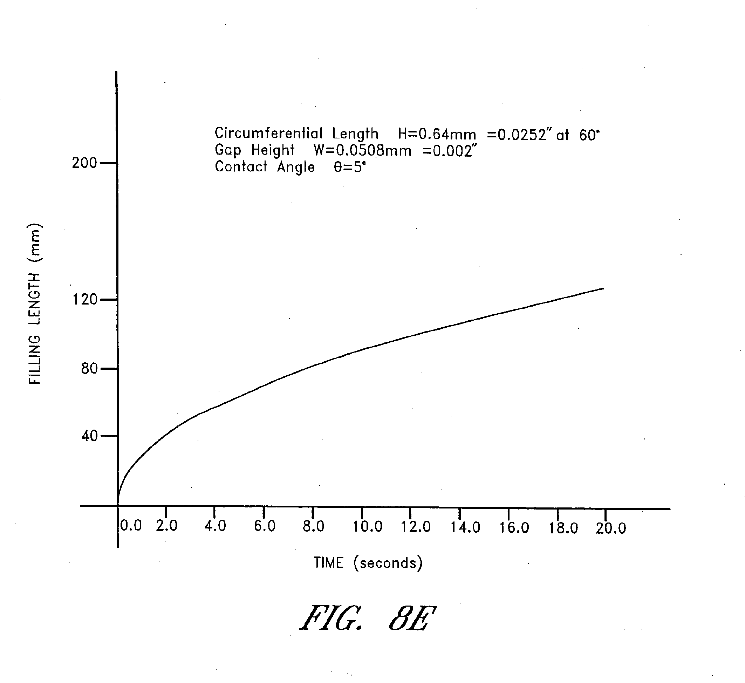

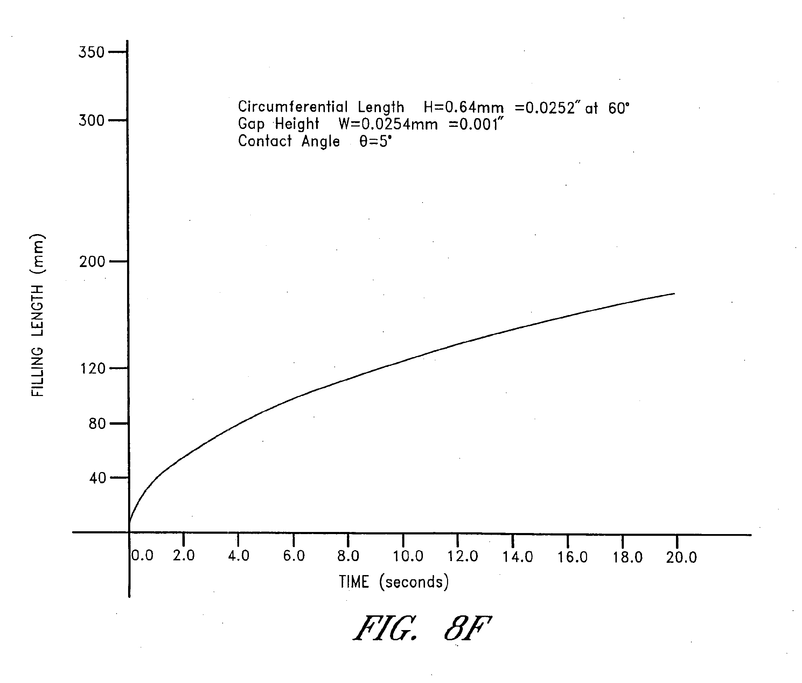

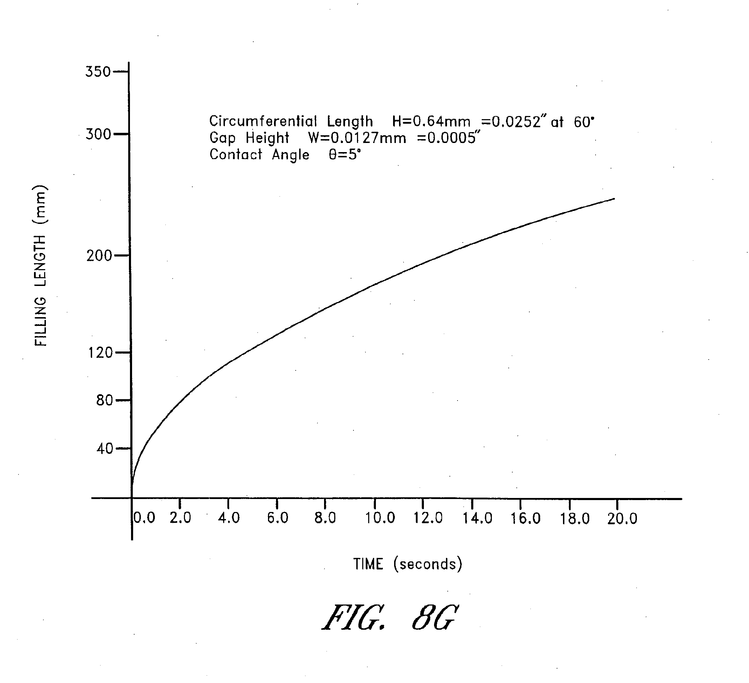

FIGURES 8E-8G are graphs of test data illustrating how quickly a fluid is drawn up the surfaces of the channel 156 when the span angle is 120 degrees, the contact angle (θ) is 5 degrees, and the circumferential length (H) is 0.64mm at 60 degrees. On each graph, the filling length (mm) is plotted on the y-axis, and time (seconds) is plotted on the x-axis. The tests were performed at hydrodynamic pressures similar to pressures experienced in peripheral vessels. FIGURE 8E illustrates the rate fluid is drawn up a channel 156 with a gap height width of 0.002 inches, FIGURE 8F illustrates the rate fluid is drawn up a channel 156 with a gap height width of 0.001 inches, and FIGURE 8G illustrates the rate fluid is drawn up a channel 156 with a gap height width of 0.0005 inches. As shown in FIGURES 8E-G, fluid is drawn up the fastest in a channel with a gap height width of 0.0005 inches, followed by a channel with a gap height width of 0.001 inches, followed by a channel with a gap height width of 0.002 inches.

The shape of the channel 156 described above and the resulting capillary action was optimized for use with whole blood as opposed to other fluids having a different viscosity than whole blood (e.g. leukocytes, pus, urine, plasma). However, the shape of the channel 156 is not limited to the disclosed shape and may be optimized for draining other liquids, such as pus. Further, the shape of the channel 156 described above was optimized for peripherally located vessels where the pressure in the vessel enhances the capillary action and resulting blood flash as well as for vessels located in the regions where the pressure may be low. For example, in the thorax region of the body, the expected pressure in the veins may be lower than in a peripherally located vein when the patient breathes. A different size of the channel for use of the access device 20 in other regions of the body may be employed taking. into account the expected pressure within the vessel or body cavity.

Additionally, an outer-surface 160 of the dilator shaft 36 and/or an inner surface 158 of the sheath body 40 can be coated with a substance to promote or enhance the capillary action within the channel 156. For example a hydrophilic substance can be used to coat outer-surface 160 of the dilator shaft 36 and/or the inner surface 158 of the sheath body 40 to enhance capillary action. As another example, a surfactant can be used to coat the outer-surface 160 of the dilator shaft 36 and the inner surface 158 of the sheath body 40. One example of a surfactant that can be used is Lutrol 68™, commercially available from BASF™; other surfactants can also be used. Other surfaces that can be coated include the inner surface of the needle body 32, the outer surface 154 of the needle body 32, the inner surface 152 of the dilator shaft 36, and the guidewire 44. These surfaces, including the outer-surface 160 of the dilator shaft 36 and the inner surface 158 of the sheath body 40, can be coated with a surfactant individually, or in combination. In the embodiments described above it may be preferable to coat both the outer-surface 160 of the dilator shaft 36 and the inner surface 158 of the sheath body 40 to promote or enhance progression of a body fluid through the channel 156. However, in some embodiments it may be preferable to only coat one of these two surfaces with a surfactant.

Use of a surfactant can accelerate and facilitate the progression of blood through the needle, dilator, or sheath. Accordingly, smaller needles, dilators, and sheaths can be used while still allowing blood to travel through said pieces with sufficient speed to indicate to an operator that the needle has entered the vessel or drainage site. Notably, in most embodiments a body fluid will pass through the needle, and thus in most embodiments it can be desirable to apply a surfactant to the interior surface of the needle.

Similarly, one or more of these components can be made of a hydrophilic material. A hydrophilic substance additionally can be applied to the outer surface of the sheath 26 to act as a lubricant to ease insertion of the sheath 26 into a patient. Other lubricants or lubricous coatings can be used on the exterior of the sheath 26 or at least the outer surface of the sheath can be formed of a lubricous material. Additionally, the sheath 26 can be coated or formed with agents (e.g., heparin), which elute from the sheath, to facilitate the clinical application of the access device 20. In one example, the outer surface of the sheath 26 can include a coating of silicone, such as Dow Corning 360 Medical Fluid, 12,5000 CST™, commercially available from Dow Coming. Similarly, the sheath can be coated with a surfactant in some embodiments.

FIGURE 8H is a cross sectional view of the embodiment depicted in FIGURE 8C along line 8H-8H. In this region of the illustrated access device 20, the sheath body 40 is coaxially positioned to minimize the annular space 157 between the sheath body 40 and the dilator shaft 36 while still allowing relative movement of the sheath body 40 and the dilator shaft 36. The inner surface 158 of the sheath body 40 need not, though it can, lie directly against the outer-surface 160 of the dilator shaft 36. The annular interface 157 between the outer-surface 160 of the dilator shaft 36 and the inner surface 158 of the sheath body 40 may be reduced in this region to inhibit the distal flow of blood or its constituents (or other bodily fluids) from the opening 74 in the dilator shaft 36.

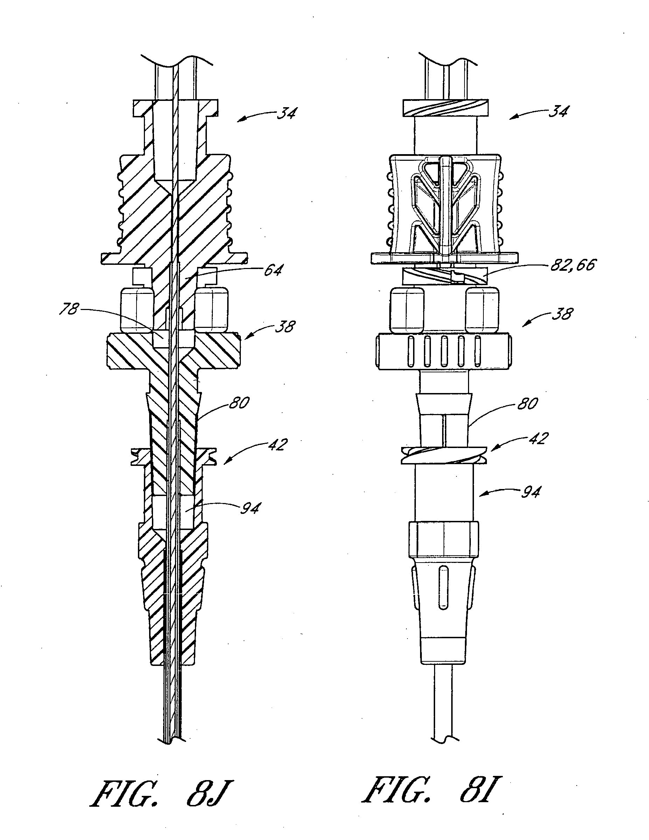

FIGURE 8I is an enlarged plan view of the portion of the embodiment illustrated in FIGURE 8A which is circled by line 8I-8I. FIGURE 8J is a cross-sectional view of the embodiment depicted in FIGURE 8I. FIGURES 8I and 8J illustrate the needle hub 34 locked to the dilator hub 38 when the needle hub is in the first position 121. The dilator shaft 36 may be coaxially mounted to the needle body 32 by slipping a hollow section 84 of the dilator shaft 36 over the needle body 32 and releasably securing the dilator hub 38 to the needle hub 34. The proximal end 86 of the dilator hub 38 is configured to mechanically fit and interlock with the needle hub 34.

The dilator shaft 36 may be releasably mounted to the needle body 32 so that the dilator shaft 36 can be mounted and released, or vice versa, from a coaxial position relative to the needle body 32. This locking mechanism can inhibit at least some unintentional rotational and axial movement between the needle 22 and the dilator 24 when the needle hub 34 is in the first position. As shown, the needle hub 34 may have a luer connection 64 that locks to the luer connection 78 of the dilator hub 38. Furthermore, the needle hub 34 may also have latch element 66 that locks to the opening 82 in the dilator hub 38.

In addition, FIGURES 8I and 8J illustrate the dilator hub 38 engaged with the sheath hub 42 when the access device 20 is inserted into a vessel 148. Preferably, the proximal end 86 of the sheath hub 42 is configured to mechanically fit and releasably engaged with the dilator hub 38. As shown, the luer connection 80 in the dilator hub 38 can engage with the lock member 94 of the sheath hub. The resulting friction fit can inhibit at least some unintentional rotational and axial movement between the dilator 24 and the sheath 26 when the access device 20 is inserted into a vessel 148.

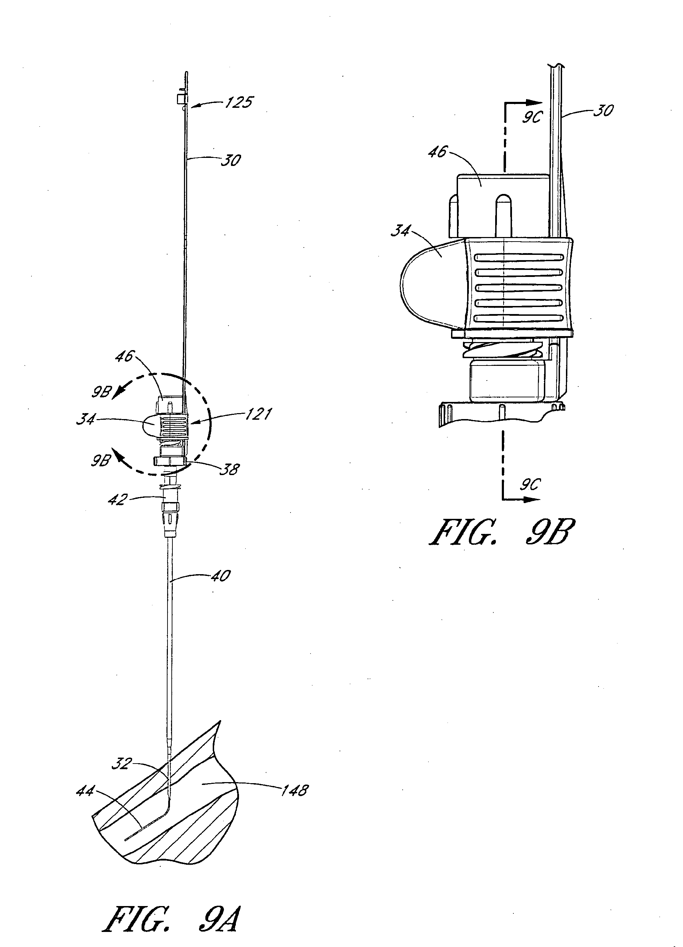

FIGURE 9A is a side view of the embodiment depicted in FIGURE 1A that illustrates a further operational step of the access device 20. FIGURE 9A depicts the guidewire 44 of the access device 20 advanced in a distal direction into a vessel 148. This can be achieved by advancing guidewire hub 46 from the third position 125 in a distal direction. The guidewire hub 46 is then locked to the needle hub 34 when the needle hub 34 is in the first position 121.

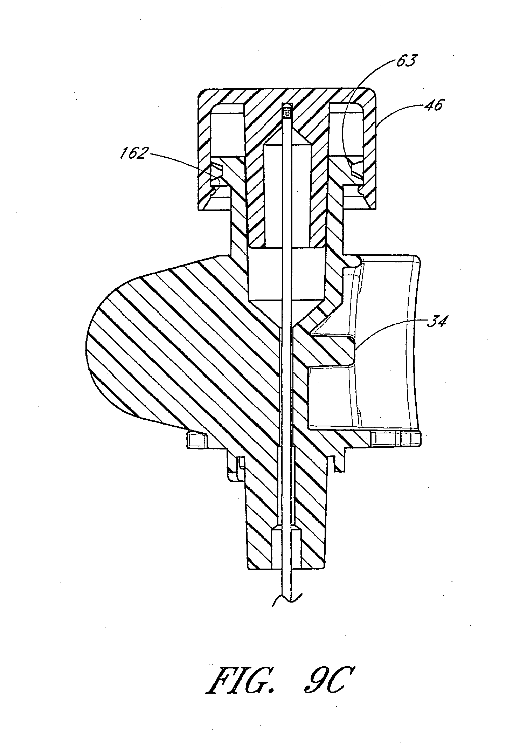

FIGURE 9B is an enlarged side view of the portion of the embodiment illustrated in FIGURE 9A which is circled by line 9B-9B. FIGURE 9C is a cross-sectional view of the embodiment depicted in FIGURE 9B. FIGURE 9C illustrates the locking mechanism between the guidewire hub 46 and the needle hub 34. Preferably, the guidewire hub 46 is configured to mechanically fit and releasably or irreversibly interlock with the needle hub 34. As shown, the guidewire hub 46 includes a nub 162 on the inner surface of the guidewire hub 46. The nub 162 of the guidewire hub can lock onto the needle hub 34 by advancing the guidewire hub 46 in a distal direction until the nub 162 is secured within the threaded groove on the lip of the needle hub 46. In other embodiments, the guidewire hub 46 can lock to the needle hub 34 via corresponding threaded elements.

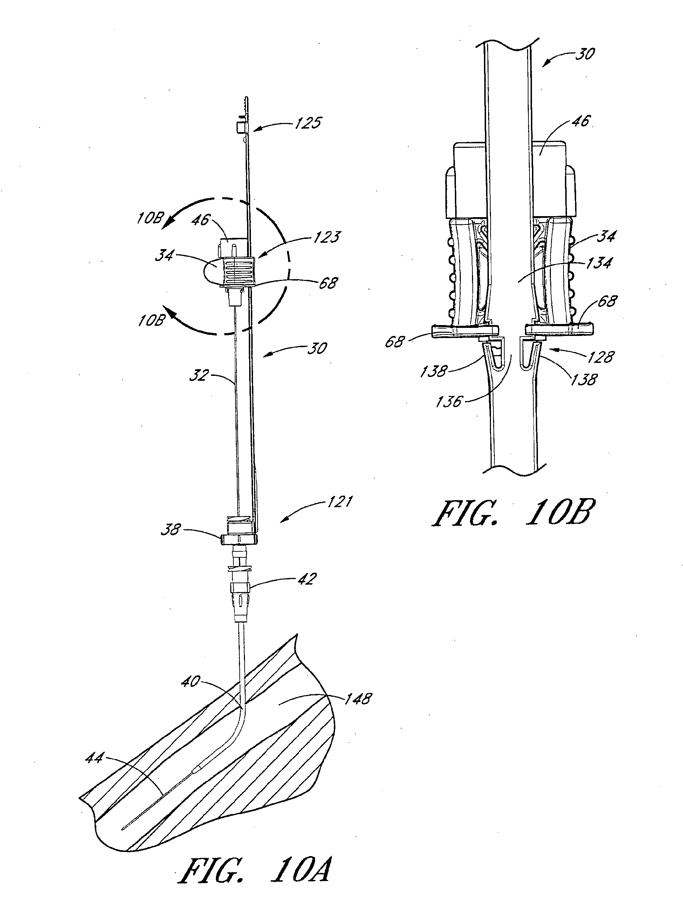

FIGURE 10A is a side view of the embodiment depicted in FIGURE 1A that illustrates another operational step of the access device 20. FIGURE 10A depicts the dilator shaft 36 and the sheath body 40 advanced in a distal direction into a vessel 148. This can be achieved by releasing the dilator hub 38 from the needle hub 34 and advancing the dilator 24 and sheath 26 in a distal direction relative to the needle hub 34 along the guidewire and needle. FIGURE 10A further illustrates the proximal movement of the needle 22 and guidewire section 28 relative to the dilator 24 and the sheath 26. The needle hub 34 will lock to the track 30 when the needle hub 36 reaches the second position 123.

FIGURE 10B is an enlarged rear view of the portion of the embodiment illustrated in FIGURE 10A which is circled by line 10B-10B. As depicted in FIGURE 10B, the needle hub 34 locks onto the track 30 via the locking mechanism 128 in the second position 123. The needle hub tangs 68 slide in a proximal direction over the track fingers 138 and the tangs 68 can lock into place between the track fingers 138 and the track section of increasing width 134. This arrests and, more preferably, substantially irreversibly prevent axial movement of the needle body 32 at least in the distal direction when the needle hub 34 is in the second position 123. In the illustrated embodiment, the locking mechanism 128 irreversibly prevents the needle hub 34 from moving in either the proximal or distal directions once engaged. Furthermore, the distal tip 54 of the needle 22 is drawn into the dilator 24 to sheath the distal tip 54 when the needle hub 34 is in the second position 123. Thus, this locking mechanism 128 inhibits the bevel tip 54 disposed on the distal portion 50 of the needle body 32 from being advanced beyond the distal end of the dilator shaft 36 once the dilator shaft 36 has been advanced over the needle body 32 during use. The dilator shaft 36 thus sheaths the sharp bevel tip 54 of the needle body 32 to inhibit accidental needle sticks from occurring.

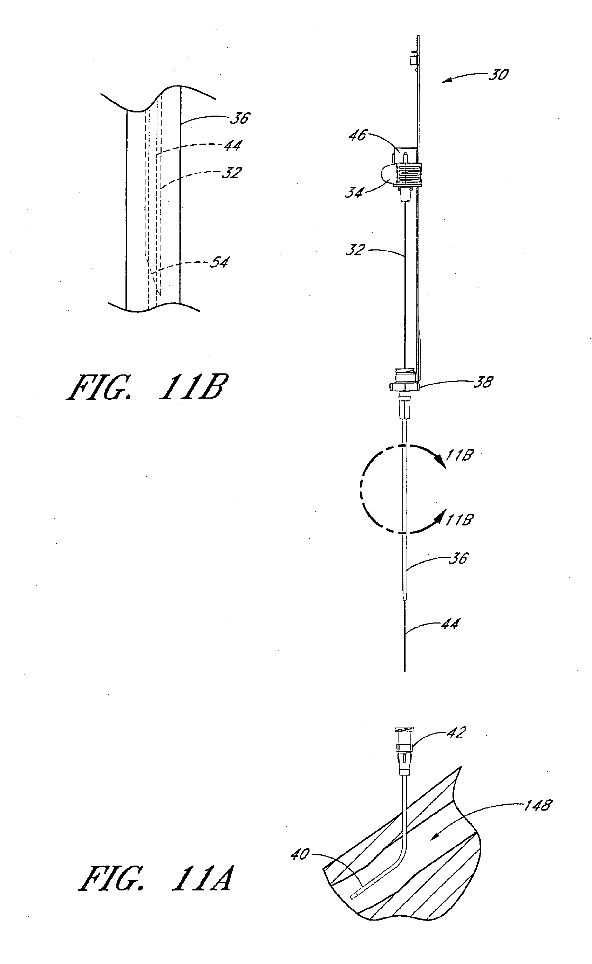

FIGURE 11A is a side view of the embodiment depicted in FIGURE 1A that illustrates the final operational step of the access device 20. FIGURE 11A illustrates the removal of the guidewire 44 and the dilator shaft 36 from the vessel leaving the sheath body 40 properly inserted within the vessel 148. FIGURE 11B is an enlarged plan view of the portion of the embodiment illustrated in FIGURE 11A which is circled by line 11B-11B. As clearly shown in FIGURE 11B, the distal end of the dilator shaft 36 and the guidewire 44 extend beyond the sharp bevel tip 54 of the needle body 32 to inhibit accidental needle sticks from occurring.

As noted above, having openings 56, 74 in the needle body 32 and dilator shaft 36 with different aspect ratios will increase the likelihood that the openings 56, 74 in the needle body 32 and dilator shaft 36 will be aligned so that blood flows substantially unobstructed through the needle side opening 56 and dilator side opening 74.

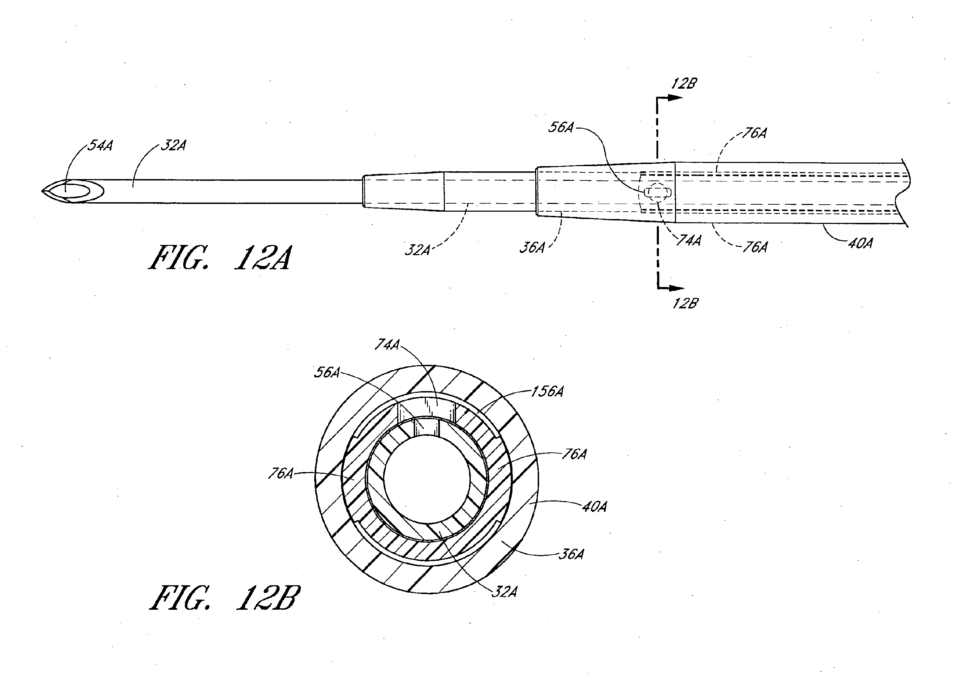

In the following embodiments, structure from one embodiment that is similar to structure from another embodiment share the same root reference number with each embodiment including a unique suffix letter (32, 32A, 32B, etc.). FIGURE 12A is a plan view of another embodiment of the openings 56, 74 in the needle body 32 and dilator shaft 36 illustrated in FIGURES 8B and 8C. FIGURE 12B is an enlarged cross-sectional view of the embodiment depicted in FIGURE 12A along line 12B-12B. FIGURES 12A and 12B depict a needle body 32A with an oblong opening 56A and a dilator shaft 36A with a circular opening 74A. In other embodiments, the needle can have a circular opening and the dilator can have an oblong opening. These embodiments can increase the likelihood that the openings 56A, 74A will be at least substantially aligned so that blood flows through the needle side opening 56A and dilator side opening 74A.

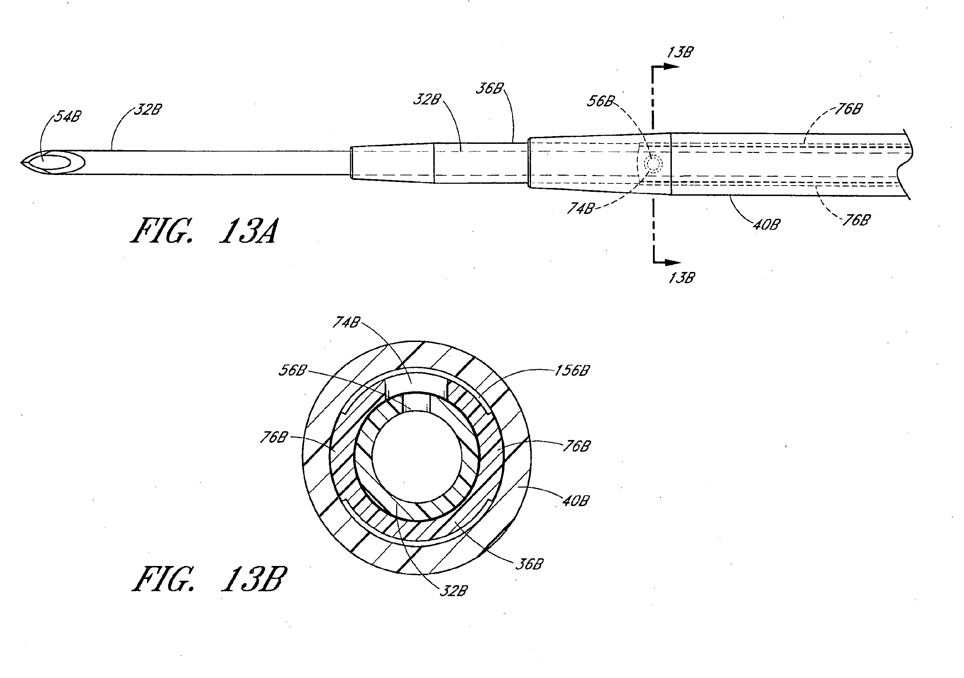

FIGURE 13A is a plan view of another embodiment of the openings 56, 74 in the needle body 32 and dilator shaft 36 illustrated in FIGURES 8B and 8C. FIGURE 13B is an enlarged cross-sectional view of the embodiment depicted in FIGURE 13A along line 13B-13B. FIGURES 13A and 13B depict a needle body 32B with a circular opening 56B and a dilator shaft 36B with a circular opening 74B that is larger than the circular opening 56B in the needle body 32B. In other embodiments, the opening in the dilator can be smaller than the opening in the needle. These embodiments can also increase the likelihood that the openings 56B, 74B will be at least substantially aligned so that blood flows through the needle side opening 56B and dilator side opening 74B.

As noted above, the dilator shaft 36 may have one or more channels 156 formed between ridges 76 to form a conduit or flow path between the sheath body 40 and the dilator shaft 36 to enable the physician or health care provider to view the blood after the bevel tip 54 of the needle body 32 has properly punctured a vessel or the channels may be formed without ridges but by extruding axial indentations of various possible configurations or by forming fully enclosed channels within the dilator shaft or body.

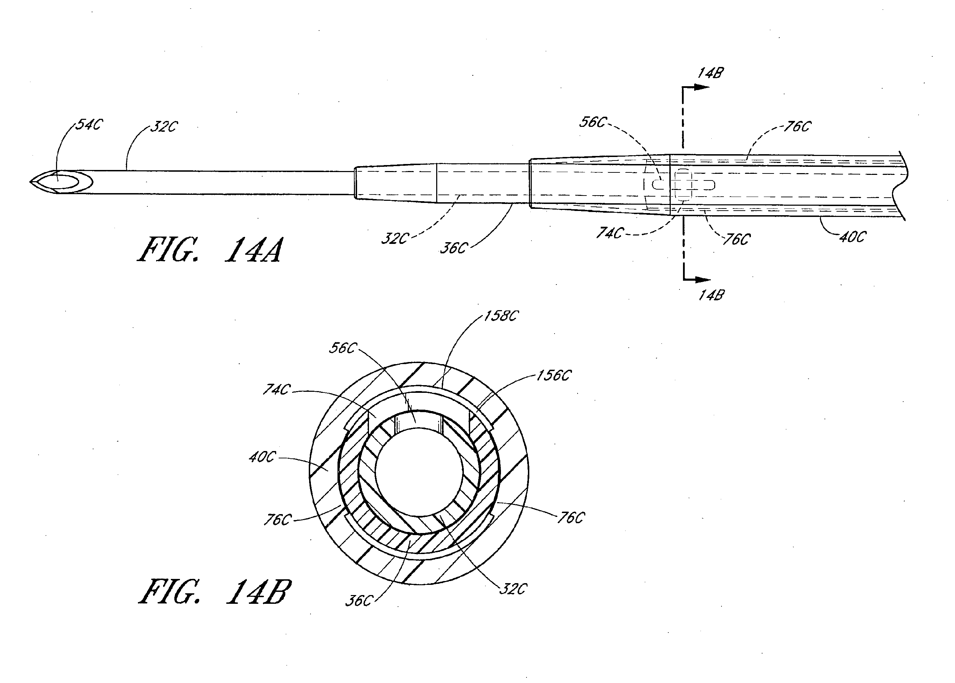

FIGURE 14A is a plan view of another embodiment of the ridges 76 depicted in FIGURE 8C. FIGURE 14B is an enlarged cross-sectional view of another embodiment of the ridges 76 depicted in FIGURE 8D. FIGURES 14A and 14B depict two ridges 76C on the inner surface 158C of the sheath body 40C that form at least one channel 156C between the sheath body 40C and the dilator shaft 36C.

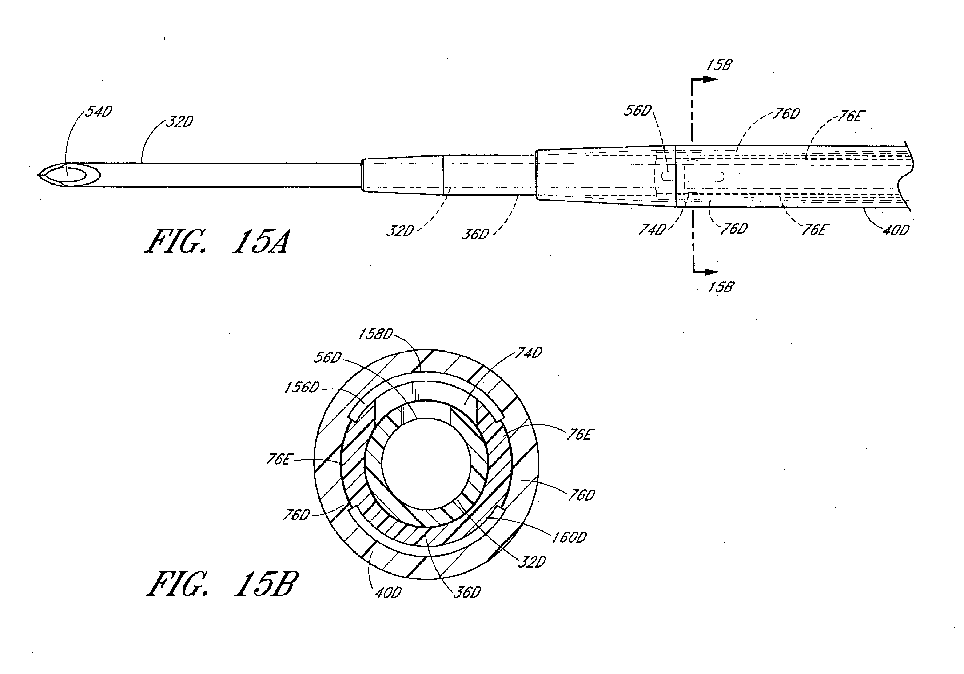

FIGURE 15A is a plan view of another embodiment of the ridges 76 depicted in FIGURE 8C. FIGURE 15B is an enlarged cross-sectional view of another embodiment of the ridges 76 depicted in FIGURE 8D. FIGURES 15A and 15B depict two ridges 76D on the inner surface 158D of the sheath body 40D and two ridges 76E on the outer surface 160D of the dilator shaft 36D that combine to form a channel 156D between the sheath body 40D and the dilator shaft 36D. For example, if the desired channel thickness is about 0.001 inches, the two ridges 76D on the inner surface 158D of the sheath body 40D can each be about 0.0005 inches thick and the two ridges 76E on the outer surface 160D of the dilator shaft 36D can each be about 0.0005 inches thick.

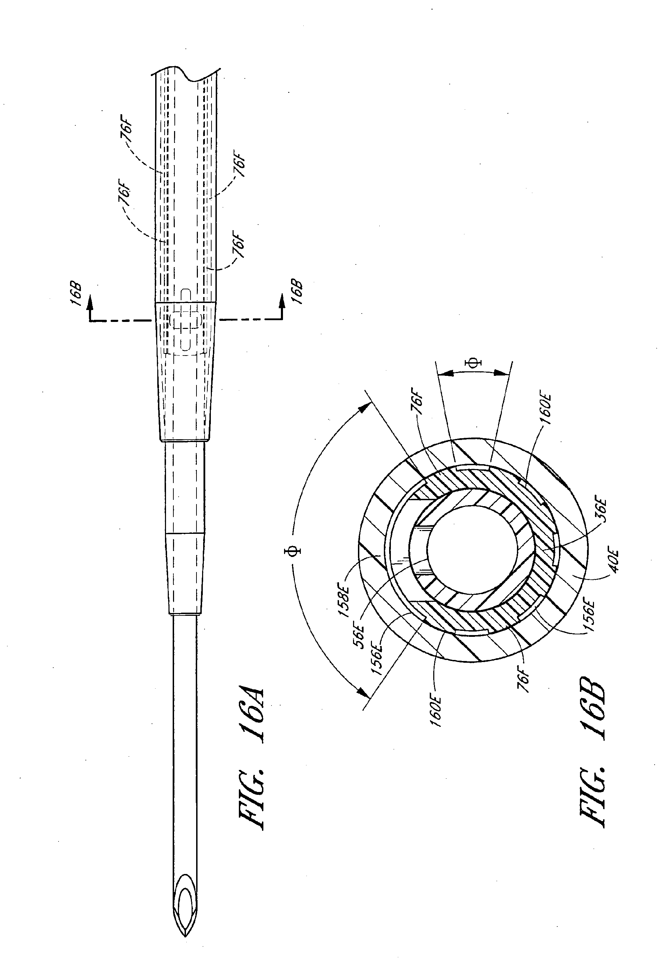

FIGURE 16A is a plan view of another embodiment of the ridges 76 depicted in FIGURE 8C. FIGURE 16B is an enlarged cross-sectional view of another embodiment of the ridges 76 depicted in FIGURE 8D. FIGURES 16A and 16B depict many ridges on the outer surface 160E of the dilator shaft 36E. Between adj acent ridges are splines 76F. The splines 76F form a plurality of channels 156E between the sheath body 40E and the dilator shaft 36E. One or more of the channels 156E can have the same span angle φ or different span angles φ. In the illustrated embodiment the channels 156E have span angles of 120 degrees and 23 degrees. In another embodiment, a single ridge 76 can spiral around the exterior of the dilator along its length.

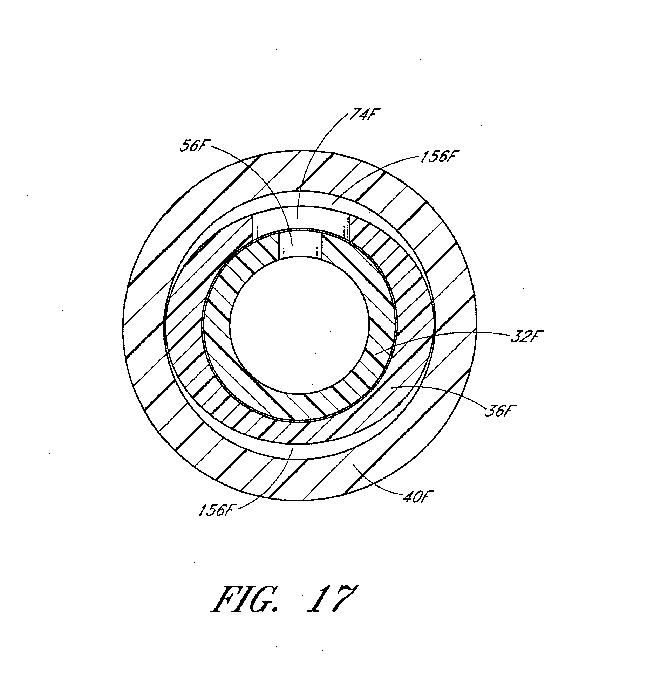

FIGURE 17 is an enlarged cross-sectional view through another embodiment of the access device and shows the channel 156F formed between a medical article or sheath body 40F and a dilator shaft 36F that have dissimilar shapes. In the illustrated embodiment, the outer surface of the dilator shaft 36F has an oval shape while the inner surface of the sheath body 40F has a round shape. The oval dilator shaft 36F and the adjacent round sheath body 40F form one or more channels or gaps 156F between the sheath body 40F and the dilator shaft 36F. Of course the shapes of the sheath body 40F and dilator shaft 36F are not limited to round and oval and may include any other combination of dissimilar shapes in adjacent regions of the sheath body 40F and dilator shaft 36F. In some modes, the outer surface of the dilator shaft 36F is oblong and the inner surface of the sheath body or medical article 40F is round. In some modes, the outer surface of the dilator shaft 36F is round and the inner surface of the medical article 40F is square. The gap or channel 156F can follow a longitudinal axis, a spiral path along the longitudinal axis, a linear path along the longitudinal axis or other path along the access device. In some modes, the linear path is parallel to the longitudinal axis. The gap or channel 156F thickness can vary along at least a portion of a length of the gap or channel 156F.

In another mode, the access device includes a blood flash-back space defined between the shaft of the needle and the shaft of the dilator. In this mode, the flash-back space preferably vents to the atmosphere and more preferably vents independent of the sheath. In particular, as described below, a vent passage is formed through the dilator, through the needle, or between the dilator and the needle.

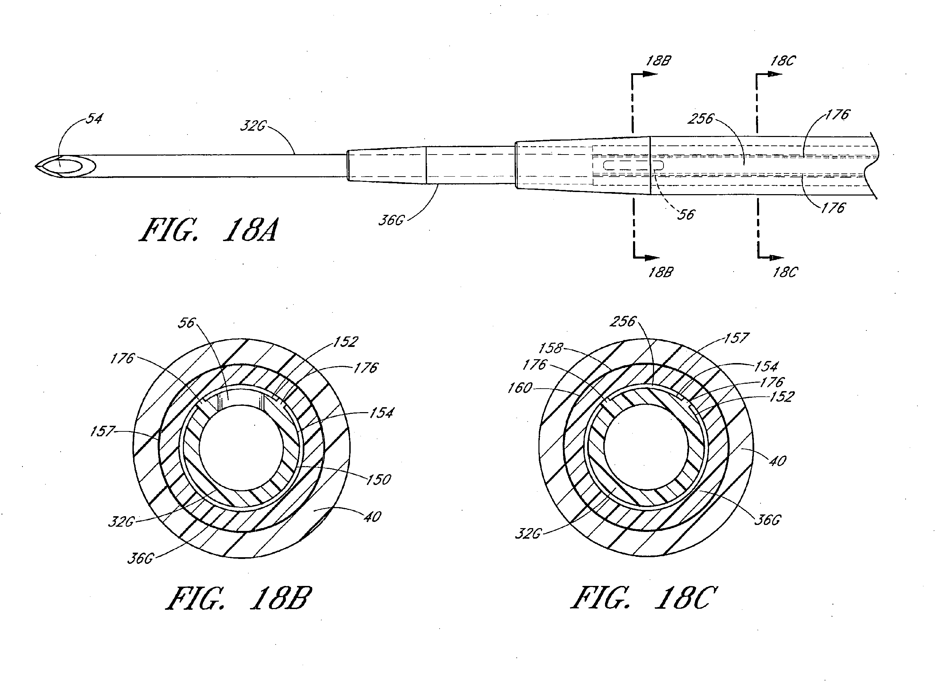

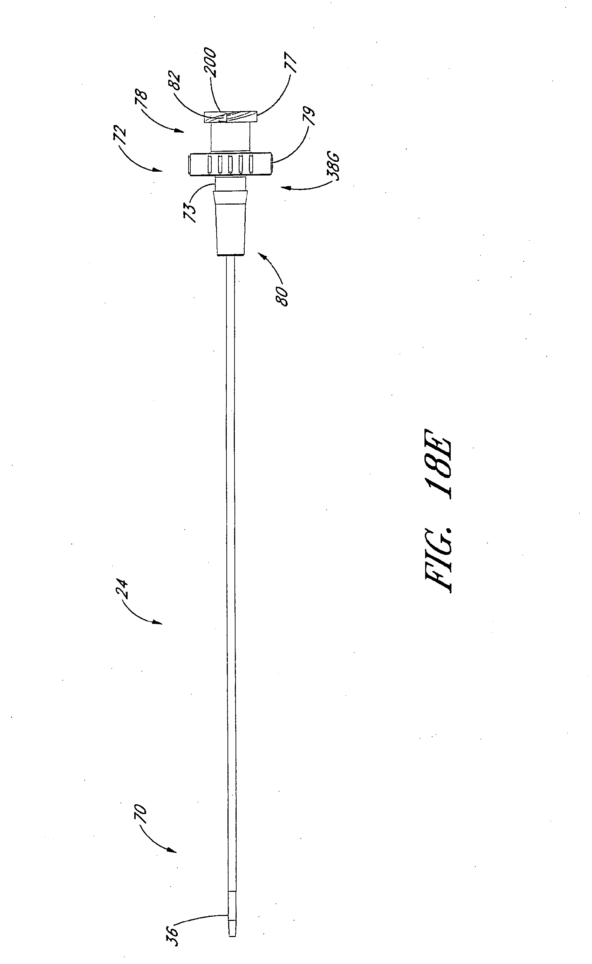

FIGURES 18A-18E illustrate an embodiment of this mode of the access device, wherein a vent channel is formed between the needle and the dilator. As best seen in FIGURES 18A-18C, the needle body 32G includes one or more fenestrations 56, and one or more ridges 176 (e.g., two ridges 176 are shown in the illustrated embodiment). The ridges 176 define the sides of at least one channel 256 extending along a length of the needle body 32G. In some embodiments additional channels 256 can be formed with additional ridges. In other embodiments channels 256 can be formed with a protruding ridge, or without a protruding ridge such as with a depression(s) or with a concentric gap. Similarly, a channel 256 can be formed with protruding or non-protruding ridges on the inner surface of the dilator shaft 36G (instead of or in addition to features on the needle body 32G). Although the channel 256 is depicted as straight, it can also form other patterns such as a helix or another shape wrapping about the access device. Further, where multiple channels are present they can form intersecting helices, parallel helices, or other patterns. In other embodiments, a distance between the needle body 32G and a dilator shaft 36G (e.g. where the inner diameter of the dilator shaft exceeds the outer diameter of the needle body) can generally define a space, or a generally annular space, similar to the space created by the channels 256.

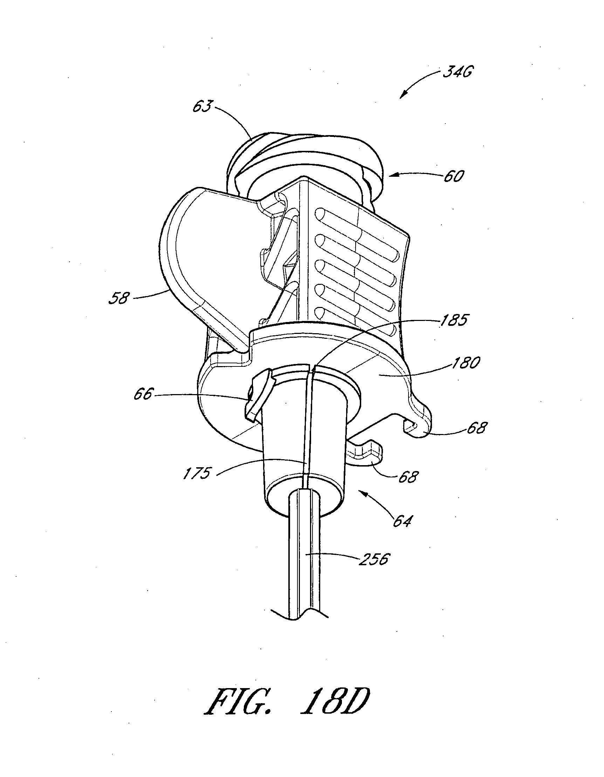

As best shown in FIGURE 18D, the needle hub 34G can include one or more venting grooves 175. As depicted, the venting grooves 175 are on the luer connection 64, but in other embodiments they can be located on the needle body 32G, on the dilator shaft 36G, pass through the needle hub 34G, pass through a dilator hub 38G, or take some other path. The venting grooves 175 can provide communication between the channels 256 (or similar spaces) and the ambient atmosphere. The luer connection 64 can be configured to cooperate with the dilator hub 38G to form a substantially liquid tight seal, such that a substance can only escape through the venting grooves 175. In embodiments where the venting grooves 175 do not extend radially, a generally radially extending side 180 of the needle hub 34G can be configured to rest far enough apart from a corresponding face 200 of the dilator hub 38G to allow air to pass between them, from the venting grooves 175.

In some embodiments,the venting grooves 175 can form a passage sufficiently small in cross-sectional area to allow the escape of gases (e.g., air) to the ambient atmosphere while hindering the escape to the ambient atmosphere of body liquids (e.g., red blood cells) with high molecular sizes, viscosities, or surface tensions. Further, in some embodiments multiple such passages can be provided, allowing adequate air ventilation despite small cross-sectional passages.

In other embodiments, the small cross-sectional area of the passage can be provided between two opposing surfaces of the dilator hub 38G and the needle hub 34G. For example, at least a portion of the venting groove 175 on the needle hub 34G can be configured to receive a generally correspondingly shaped venting surface on the dilator hub 38G without entirely blocking the venting groove. The resulting passage between the surfaces of the needle hub 34G and the dilator hub 38G thus define at least a region of relatively small cross-sectional area to permit air flow but restrict the flow of bodiy fluids.

While the venting structure is depicted as grooves 175 in the illustrated embodiment, other structures can perform similar functions. For example, a single reduced space location between the needle body 32G and the dilator body 34G can permit the escape of air while inhibiting the flow of blood proximally beyond the reduced space location. Similarly, a labyrinth passage can be disposed between the ambient atmosphere and the flash-back space (the space between the needle and dilator).

In other embodiments, one or more of the venting grooves 175 can be filled at least in part by a porous material that permits gases to flow through the material but inhibits the passage of a body fluid (e.g., blood). Such material can be integrally formed into the needle hub 34G or dilator hub 38G such that the material and the hubs are unitary. The material can then comprise any portion of the length of the venting grooves 175. In other embodiments the material can be placed into the venting grooves 175 or a receptacle in communication with the groove(s). When the material is placed into the groove 175, the groove can include a receiving portion such as a groove notch 185 configured to receive the porous material. One or more of the vent passages in other embodiments can be entirely formed by such porous material. Suitable porous materials include, but are not limited to a porous polymer such as HDPE, UHMWPE, PP, PTFE, PVDF, EVA, PE, Nylon, and PU, of pore size approximately 2.5 microns. In further embodiments, a combination of pore volume and pore size can be chosen to allow passage of gases (such as air) but inhibit the passage of body fluids (such as blood).

In further embodiments, the venting passages can be tubes defined solely by either the needle hub 34G or the dilator hub 38G. For example, the channel 256 can lead to an opening in the needle hub 34G. This opening can include any of the characteristics discussed above to control the passage of gases and fluids. The opening can thus allow the escape of gases (e.g. air) through the needle hub 34G to the ambient atmosphere while inhibiting the passage of body fluids (e.g. blood). In other embodiments, a similar venting passage can be a tube defined solely by the dilator hub 38G. It will be clear from the disclosure herein that a variety of passages (e.g. venting grooves 175, tubes, porous material, etc.) can be used to allow the escape of gases (e.g. air) to the ambient atmosphere while inhibiting the escape of body fluids (e.g. blood).

In another embodiment, the venting passages can be within the dilator shaft 36G and the sheath body 40. For example, a venting hole or a patch of venting material can be provided in each of the dilator shaft 36G and the sheath body 40. In some embodiments these venting structures can overlap, allowing gases to pass directly from one to the other. In other embodiments, these venting structures can be positioned some distance away from each other, in which case a channel or groove similar to those in FIGURE 18D can be provided between the dilator shaft 36G and the sheath body 40 to bring the venting structures into communication. These venting structures can be provided proximal from the fenestration 56 in the needle body 32G.

As shown, the dilator shaft 36G in this embodiment can have no fenestration and can be generally continuous. The dilator shaft 36G can thus radially close the channel 256 (or similar space). In similar embodiments the same functionality can be accomplished with ridges in the dilator shaft 36G cooperating with an otherwise generally continuous needle 32G including a fenestration 56. The dilator shaft 36G can be formed of a translucent material in the entirety, or alternatively be translucent in at least the region adjacent the channel 256. The sheath body 40 can be similarly formed of a translucent material. In other embodiments, the material can be transparent instead of only translucent. In further embodiments, the material can be only partially translucent both spatially and temporally. Spatially, the material of the dilator shaft 36G and/or the sheath body 40 can be translucent near the channel 256, allowing visual confirmation of e.g. blood flash-back. Temporally, the visual characteristics of the material can change upon entry of a body fluid (e.g. due to temperature change or molecular interaction). The material can thus become translucent upon entry of a body fluid, or in other embodiments change color or provide some other visual indication.

Further, the access device depicted in FIGURES 18A-18E can include surfactants and/or lubricious coatings, as described above. For example, in some embodiments a surfactant can be applied to the interior of the dilator shaft 36G, the exterior of the needle 32G, and/or the interior of the needle. The surfactant can be applied to any combination of these surfaces, depending on the desired effect. For example, the surfactant can be applied solely to the outer surface of the needle, solely to the inner surface of the dilator, or solely to the inner surface of the needle. As another example, a surfactant can be applied to combinations of these surfaces, such as to both the inner surface of the dilator and the outer surface of the needle. The surfactant can ease the passage of a body fluid through spaces within the access device, accelerating flashback. As another example, in some embodiments a similar channel can be provided between a dilator shaft and a sheath body, and the surfactant can be supplied on the inner surface of the sheath and the outer surface of the dilator. Even further, in some embodiments channels can be provided both between the dilator and needle and the dilator and sheath, with the channels being in communication via a fenestration in the dilator, as described herein. Furher, as described above, the outer surface of the sheath can be coated with a surfactant, lubricious material, or the like.

In other embodiments, the channel 156 can be formed by having one complete ridge on the inner surface of the sheath and one complete ridge on the outer surface of the dilator. In other embodiments, the inner surface of the sheath can have two ridges that run 50% of the length of the channel 156 and the outer surface of the dilator can have two ridges that run the remaining 50% of the channel 156.

The embodiments herein described are comprised of conventional, biocompatible materials. For example, the needle preferably consists of ceramic, a rigid polymer, or a metal such as stainless steel, nitinol, or the like. The other elements can be formed of suitable polymeric materials, such as polycarbonate, nylon, polyethylene, high-density polyethylene, polypropylene, fluoropolymers and copolymers such as perfluoro (ethylene-propylene) copolymer, polyurethane polymers or co-polymers.

As noted above, the present access device can be used to place a catheter at other locations within a patient's body. Thus, for example, but without limitation, the access device can be used as or with a variety of catheters to drain fluids from abscesses, to drain air from a pneumotorax, and to access the peritoneal cavity. In such applications, body fluids flow into the viewing space to indicate when the needle has been properly placed.

Although this invention has been disclosed in the context of certain preferred embodiments and examples, it will be understood by those skilled in the art that the present invention extends beyond the specifically disclosed embodiments to other alternative embodiments and/or uses of the invention and obvious modifications and equivalents thereof. In addition, while a number of variations of the invention have been shown and described in detail, other modifications, which are within the scope of this invention, will be readily apparent to those of skill in the art based upon this disclosure. It is also contemplated that various combinations or sub-combinations of the specific features and aspects of the embodiments may be made and still fall within the scope of the invention. Accordingly, it should be understood that various features and aspects of the disclosed embodiments can be combined with or substituted for one another in order to form varying modes of the disclosed invention. For example, the general shape of the needle hub depicted in Figure 18D differs in additional ways from the needle hub depicted in Figure 2F. However, these general needle hub shapes can be interchanged between the described and depicted embodiments. Thus, it is intended that the scope of the present invention herein disclosed should not be limited by the particular disclosed embodiments described above, but should be determined only by a fair reading of the disclosure and the claims that follow.

The following aspects are also part of the present invention:
Aspect 1: An access device for placing a medical article within a body space, comprising: a needle having an elongated needle body with a distal end and a hub from which the needle body extends, the needle body comprising an inner surface, an outer surface, and a side hole; a dilator disposed on the needle body, the dilator comprising a dilator body and a dilator hub, the dilator body comprising an inner surface and an outer surface; and a sheath disposed on the dilator body, the sheath comprising a sheath body and a sheath hub, the sheath body comprising an inner surface and an outer surface, wherein at least one of the outer surface of the needle, inner surface of the dilator, outer surface of the dilator, and inner surface of the sheath is coated at least partially with a surfactant or a lubricious material, and wherein a space is defined somewhere between the inner surface of the sheath and the outer surface of the needle, the space being in communication with the side hole.
Aspect 2: The access device of aspect 1, wherein the inner surface of the needle is coated at least partially with a surfactant.
Aspect 3: The access device of any of aspects 1 and 2, wherein the outer surface of the needle is coated at least partially with a surfactant.
Aspect 4: The access device of any of the preceding aspects, wherein the inner surface of the dilator is coated at least partially with a surfactant.
Aspect 5: The access device of any of the preceding aspects, wherein the outer surface of the dilator is coated at least partially with a surfactant.
Aspect 6: The access device of any of the preceding aspects, wherein the inner surface of the sheath is coated at least partially with a surfactant.
Aspect 7: The access device of any of the preceding aspects, wherein the outer surface of the sheath is coated at least partially with a lubricious material.
Aspect 8: The access device of any of the preceding aspects, further comprising a vent in at least one of the dilator or sheath.
Aspect 9: The access device of aspect 8, wherein the vent allows the egress of air and hinders the egress of a fluid to the ambient atmosphere.
Aspect 10: The access device of aspect 9, wherein the vent comprises a porous material.
Aspect 11. The access device of aspect 9, wherein the vent comprises a narrow channel.
Aspect 12: An access device for placing a medical article within a body space, comprising: a needle having an elongated needle body with a distal end and a hub from which the needle body extends; a dilator disposed on the needle body, the needle and the dilator being moveable relative to each other from a first position, wherein the distal end of the needle lies distal of the dilator, and a second position wherein the distal end of the needle lies within the dilator, the dilator including a dilator hub and an elongated dilator shaft that extends from the dilator hub; a locking mechanism operating between the needle and the dilator to inhibit movement of the needle relative to the dilator when in the second position, the locking mechanism being configured to allow movement of the needle from the first position toward the second position without engagement by the locking mechanism so as to lessen resistance to the movement; and a sheath disposed on the dilator, the sheath and the dilator being moveable relative to each other, wherein at least one of the outer surface of the needle, inner surface of the dilator, outer surface of the dilator, and inner surface of the sheath is coated at least partially with a surfactant or a lubricious material.
Aspect 13: The access device of aspect 12, wherein the inner surface of the sheath and/or the outer surface of the dilator is coated at least partially with a surfactant or a lubricious material.
Aspect 14: The access device of either aspect 12 or 13, wherein the inner surface of the dilator and/or the outer surface of the needle is coated at least partially with a surfactant or a lubricious material.
Aspect 15: The access device of any of aspects 12-14, wherein the outer surface of the sheath is coated at least partially with a surfactant or a lubricious material.
Aspect 16: The access device of any of aspects 12-15, wherein the inner surface of the sheath is coated at least partially with a surfactant or a lubricious material.
Aspect 17: The access device of any of aspects 12-16, wherein the outer surface of the dilator is coated at least partially with a surfactant or a lubricious material.
Aspect 18: The access device of any of aspects 12-17, wherein the inner surface of the dilator is coated at least partially with a surfactant or a lubricious material.
Aspect 19: The access device of any of aspects 12-18, wherein the outer surface of the needle is coated at least partially with a surfactant or a lubricious material.
Aspect 20: The access device of any of aspects 12-19, wherein the inner surface of the needle is coated at least partially with a surfactant or a lubricious material.
Aspect 21: The access device of any of aspects 12-20, further comprising a vent in at least one of the dilator or sheath.
Aspect 22: The access device of aspect 21, wherein the vent allows the egress of air and hinders the egress of a fluid to the ambient atmosphere.
Aspect 23: The access device of aspect 22, wherein the vent comprises a porous material.
Aspect 24: The access device of aspect 22, wherein the vent comprises a narrow channel.
Aspect 25: The access device of aspect 12, wherein a longitudinal length of the needle is greater than a longitudinal length of the dilator, and a distance between the first position and the second position is less than a longitudinal length of the needle.
Aspect 26: The access device of aspect 12, wherein the sheath is configured to receive at least a portion of a catheter.
Aspect 27: The access device of aspect 12, wherein the locking mechanism comprises a first member supported on the proximal side of and spaced from the dilator hub and a second member supported by the needle, the first and second members being configured to engage each other when the needle hub is moved through a distance away from the dilator hub, said distance being greater than a distance by which the distal end of the needle body extends beyond the dilator when the hubs of the needle and dilator are juxtaposed.
Aspect 28: The access device of aspect 27, wherein an elongated support suspends the first member of the locking mechanism at a location spaced from the dilator hub.
Aspect 29: The access device of aspect 28, wherein the elongated support and the needle hub include cooperating structure such that the needle slides along the elongated support.
Aspect 30: The access device of aspect 29, wherein the elongated support is rotatably coupled to the dilator hub.
Aspect 31: The access device of aspect 28, wherein the elongated support lies generally parallel to a longitudinal axis of the needle body.
Aspect 32: The access device of aspect 28, wherein the elongated support extends to the proximal side of the dilator hub.
Aspect 33: The access device of aspect 28 additionally comprising a guidewire extending distally from a guidewire cap, the guidewire cap being releasably coupled to the elongated support.
Aspect 34: The access device of aspect 33, wherein the guidewire cap engages the elongated support at a location proximate of the first member of the locking mechanism.
Aspect 35: The access device of any of aspects 12-34, wherein the needle body includes at least one fenestration and the dilator shaft includes at least one fenestration that communicates with the needle fenestration.
Aspect 36: The access device of aspect 35, wherein one of said fenestrations has a greater dimension in at least one direction than the other one of said fenestrations.
Aspect 37: The access device of aspect 35 wherein the sheath is coaxially disposed about the dilator shaft, and at least one elongated channel is formed between the dilator shaft and the sheath that extends along at least a substantial portion of the length of the dilator shaft, the channel communicating with the fenestration in the dilator shaft and having a dimension, in a direction perpendicular to the length of the dilator shaft, that is less than a maximum outer diameter of the dilator shaft.
Aspect 38: The access device of aspect 35 wherein the sheath is coaxially disposed about the dilator shaft, and at least one elongated channel is formed between the dilator shaft and the sheath that extends along at least a substantial portion of the length of the dilator shaft, the channel communicating with the fenestration in the dilator shaft and having a span angle of less than 360 degrees about a longitudinal axis of the dilator.
Aspect 39: An access device for placing a medical article within a body space, comprising: a needle including a needle body having a longitudinal axis with a distal tip and a needle hub from which the needle body extends; a dilator including a dilator shaft and a dilator hub, the dilator shaft being disposed on and slideable along the needle body with the dilator hub being disposed distal of the needle hub; a sheath comprising a tubular section and a hub, the tubular section being disposed on and slideable along the dilator with the hub being disposed distal of the dilator hub; a track extending from the dilator hub in a proximal direction; and a locking mechanism operably disposed between the track and the needle so as to selectively inhibit proximal movement of the needle relative to the dilator, wherein at least one of the outer surface of the needle, inner surface of the dilator, outer surface of the dilator, and inner surface of the sheath is coated at least partially with a surfactant or a lubricious material.
Aspect 40. The access device of aspect 39, wherein the inner surface of the sheath and/or the outer surface of the dilator is coated at least partially with a surfactant or a lubricious material.
Aspect 41: The access device of either aspect 39 or 40, wherein the inner surface of the dilator and/or the outer surface of the needle is coated at least partially with a surfactant or a lubricious material.
Aspect 42: The access device of any of aspects 39-41, wherein the outer surface of the sheath is coated at least partially with a surfactant or a lubricious material.
Aspect 43. The access device of any of aspects 39-42, wherein the inner surface of the sheath is coated at least partially with a surfactant or a lubricious material.
Aspect 44: The access device of any of aspects 39-43, wherein the outer surface of the dilator is coated at least partially with a surfactant or a lubricious material.
Aspect 45: The access device of any of aspects 39-44, wherein the inner surface of the dilator is coated at least partially with a surfactant or a lubricious material.
Aspect 46: The access device of any of aspects 39-45, wherein the outer surface of the needle is coated at least partially with a surfactant or a lubricious material.
Aspect 47: The access device of any of aspects 39-46, wherein the inner surface of the needle is coated at least partially with a surfactant or a lubricious material.
Aspect 48: The access device of any of aspects 39-47, further comprising a vent in at least one of the dilator or sheath.
Aspect 49: The access device of aspect 48, wherein the vent allows the egress of air and hinders the egress of a fluid to the ambient atmosphere.
Aspect 50: The access device of aspect 49, wherein the vent comprises a porous material.
Aspect 51: The access device of aspect 49, wherein the vent comprises a narrow channel.
Aspect 52: The access device of aspect 39, wherein the needle comprises at least one tang engaging the track.
Aspect 53: The access device of aspect 39, wherein the track is rotatable around the longitudinal axis of the dilator hub.
Aspect 54: The access device of aspect 39, wherein the locking mechanism comprises at least one finger element projecting from the track.
Aspect 55: The access device of aspect 39, wherein a width of the track varies along its longitudinal length.
Aspect 56. The access device of aspect 39, wherein the needle is configured to slide along at least a portion of the track between a first position and a second position, the second position being on a proximal side of the first position, the locking mechanism inhibiting further proximal movement of the needle when the needle is in the second position.
Aspect 57: The access device of aspect 56, wherein a longitudinal length of the dilator is sufficiently long so that the distal tip of the needle body lies within the dilator at least when the needle is in the second position.
Aspect 58: The access device of aspect 56, wherein the locking mechanism comprises a slot in the track, the slot being arranged perpendicular to the longitudinal axis and being configured to receive the at least one tang of the needle at least when the needle is in the second position.
Aspect 59: The access device of aspect 39 further comprising a guidewire, the needle body and the guidewire being coaxially disposed with the guidewire slideable through at least a portion of the needle body.
Aspect 60: The access device of aspect 59, wherein the guidewire comprises a wire and a hub, the hub being configured to engage with the needle hub.
Aspect 61: The access device of aspect 60 further comprising a second locking mechanism operably disposed between the track and the guidewire hub, the second locking mechanism being configured to selectively release the guidewire hub from the track so as to allow the guidewire hub to move in a distal direction as the guidewire slides through the needle body.
Aspect 62: The access device of aspect 61, wherein the second locking mechanism comprises: an element protruding from the track and configured to inhibit longitudinal movement of the hub in a proximal direction relative to the track; at least one arm projecting from the track on a distal side of the element, the at least one arm being configured to selectively inhibit movement of the hub in at least a lateral direction relative to the track; and a stop protruding from the track on a distal side of the at least one arm, the stop being configured to selectively inhibit longitudinal movement of the hub in the distal direction relative to the track.
Aspect 63: The access device of aspect 39, wherein the track is rotatably coupled to the dilator.
Aspect 64: The access device of aspect 39, wherein a third locking mechanism operates between the needle hub and the dilator hub.
Aspect 65: The access device of aspect 64, wherein the third locking mechanism is unlocked by relative rotational movement between the needle and dilator hubs, and wherein the needle hub includes a longitudinal tab that projects above an outer surface of the dilator hub.
Aspect 66: The access device of aspect 65, wherein the needle body includes a beveled distal tip and the tab is located on a side of the needle hub that corresponds with the side of the needle body on which the bevel is formed.
Aspect 67. An access device for placing a medical article within a body space, comprising: a needle having a distal end and a first fenestration; a dilator disposed on and slideable along the needle and having a second fenestration, wherein one of the first and second fenestrations has a greater dimension in at least one direction than the other one of the first and second fenestrations in said direction; and a sheath being coaxially disposed and longitudinally movable over the dilator, wherein at least one of the outer surface of the needle, inner surface of the dilator, outer surface of the dilator, and inner surface of the sheath is coated at least partially with a surfactant or a lubricious material.
Aspect 68: The access device of aspect 67, wherein the inner surface of the sheath and/or the outer surface of the dilator is coated at least partially with a surfactant or a lubricious material.
Aspect 69: The access device of either aspect 67 or 68, wherein the inner surface of the dilator and/or the outer surface of the needle is coated at least partially with a surfactant or a lubricious material.
Aspect 70: The access device of any of aspects 67-69, wherein the outer surface of the sheath is coated at least partially with a surfactant or a lubricious material.
Aspect 71: The access device of any of aspects 67-70, wherein the inner surface of the sheath is coated at least partially with a surfactant or a lubricious material.
Aspect 72: The access device of any of aspects 67-71, wherein the outer surface of the dilator is coated at least partially with a surfactant or a lubricious material.
Aspect 73: The access device of any of aspects 67-72, wherein the inner surface of the dilator is coated at least partially with a surfactant or a lubricious material.
Aspect 74: The access device of any of aspects 67-73, wherein the outer surface of the needle is coated at least partially with a surfactant or a lubricious material.
Aspect 75: The access device of any of aspects 67-74, wherein the inner surface of the needle is coated at least partially with a surfactant or a lubricious material.
Aspect 76: The access device of any of aspects 67-75, further comprising a vent in at least one of the dilator or sheath.
Aspect 77: The access device of aspect 76, wherein the vent allows the egress of air and hinders the egress of a fluid to the ambient atmosphere.
Aspect 78: The access device of aspect 77, wherein the vent comprises a porous material.
Aspect 79: The access device of aspect 77, wherein the vent comprises a narrow channel.
Aspect 80: The access device of aspect 67, wherein the at least one direction is a longitudinal direction.
Aspect 81: The access device of aspect 67, wherein the at least one direction is circumferential.
Aspect 82: The access device of aspect 67, wherein a shape of the first fenestration is oblong and a shape of the second fenestration is round.
Aspect 83: The access device of aspect 67, wherein a shape of each of the first and second fenestrations is oblong.
Aspect 84: The access device of aspect 67, wherein the first oblong fenestration is arranged generally perpendicular to the second oblong fenestration.
Aspect 85: An access device for placing a medical article within a body space, comprising: a needle having a distal end and at least one fenestration; a dilator including a shaft disposed on at least a portion of the needle; a sheath disposed on at least a portion of the dilator; and at least one elongated channel disposed between the needle and an exterior surface of the sheath that extends along at least a substantial portion of the length of the dilator shaft, the channel communicating with the fenestration in the needle and having a span angle of less than 360 degrees about a longitudinal axis of the dilator, wherein at least one of the outer surface of the needle, inner surface of the dilator, outer surface of the dilator, and inner surface of the sheath is coated at least partially with a surfactant or a lubricious material.
Aspect 86: The access device of aspect 85, wherein the inner surface of the sheath and/or the outer surface of the dilator is coated at least partially with a surfactant or a lubricious material.
Aspect 87: The access device of either aspect 85 or 86, wherein the inner surface of the dilator and/or the outer surface of the needle is coated at least partially with a surfactant or a lubricious material.
Aspect 88: The access device of any of aspects 85-87, wherein the outer surface of the sheath is coated at least partially with a surfactant or a lubricious material.
Aspect 89: The access device of any of aspects 85-88, wherein the inner surface of the sheath is coated at least partially with a surfactant or a lubricious material.
Aspect 90: The access device of any of aspects 85-89, wherein the outer surface of the dilator is coated at least partially with a surfactant or a lubricious material.
Aspect 91: The access device of any of aspects 85-90, wherein the inner surface of the dilator is coated at least partially with a surfactant or a lubricious material.
Aspect 92: The access device of any of aspects 85-91, wherein the outer surface of the needle is coated at least partially with a surfactant or a lubricious material.
Aspect 93: The access device of any of aspects 85-92, wherein the inner surface of the needle is coated at least partially with a surfactant or a lubricious material.
Aspect 94: The access device of any of aspects 85-93, further comprising a vent in at least one of the dilator or sheath.
Aspect 95: The access device of aspect 94, wherein the vent allows the egress of air and hinders the egress of a fluid to the ambient atmosphere.
Aspect 96: The access device of aspect 95, wherein the vent comprises a porous material.
Aspect 97: The access device of aspect 95, wherein the vent comprises a narrow channel.
Aspect 98:The access device of aspect 85, wherein the dilator comprises a second fenestration, the second fenestration communicating with the fenestration in the needle.
Aspect 99: The access device of aspect 98, wherein at least a portion of the second fenestration aligns with a portion of the fenestration in the dilator.
Aspect 100: The access device of aspect 85, wherein the channel is disposed between the dilator and the sheath.
Aspect 101: The access device of aspect 100, wherein the channel is defined at least in part by at least one longitudinal groove formed on an outer surface of the dilator shaft.
Aspect 102: The access device of aspect 100, wherein the groove has a span angle of around 120 degrees.
Aspect 103: The access device of aspect 100, wherein at least a portion of the groove has a radial dimension of approximately 0.001 inches.
Aspect 104: The access device of aspect 100, wherein the longitudinal groove follows a spiral path along at least a portion of the length of the dilator shaft.
Aspect 105: The access device of 100, additionally comprising a second longitudinal groove diametrically opposed to said other groove on an outer surface of the dilator shaft.
Aspect 106: The access device of aspect 100, wherein the groove has a longitudinal length less than a length of the dilator shaft.
Aspect 107: The access device of aspect 100, wherein the distal end of the groove lies proximal of a distal end of the dilator.
Aspect 108: An access device for placing a medical article within a body space, comprising: a needle having a distal end and a longitudinal axis; a dilator disposed on at least a portion of the needle and having an outer surface; and a sheath disposed on at least a portion of the dilator and having an inner surface, at least a portion of the inner surface of the sheath having a dissimilar shape than an adjacent portion of the outer surface of the dilator so as to form a gap therebetween, the gap extending along the longitudinal axis, wherein at least one of the outer surface of the needle, inner surface of the dilator, outer surface of the dilator, and inner surface of the sheath is coated at least partially with a surfactant or a lubricious material.
Aspect 109: The access device of aspect 108, wherein the inner surface of the sheath and/or the outer surface of the dilator is coated at least partially with a surfactant or a lubricious material.
Aspect 110: The access device of either aspect 108 or 109, wherein the inner surface of the dilator and/or the outer surface of the needle is coated at least partially with a surfactant or a lubricious material.
Aspect 111: The access device of any of aspects 108-110, wherein the outer surface of the sheath is coated at least partially with a surfactant or a lubricious material.
Aspect 112: The access device of any of aspects 108-111, wherein the inner surface of the sheath is coated at least partially with a surfactant or a lubricious material.
Aspect 113: The access device of any of aspects 108-112, wherein the outer surface of the dilator is coated at least partially with a surfactant or a lubricious material.
Aspect 114: The access device of any of aspects 108-113, wherein the inner surface of the dilator is coated at least partially with a surfactant or a lubricious material.
Aspect 115: The access device of any of aspects 108-114, wherein the outer surface of the needle is coated at least partially with a surfactant or a lubricious material.
Aspect 116: The access device of any of aspects 108-115, wherein the inner surface of the needle is coated at least partially with a surfactant or a lubricious material.
Aspect 117: The access device of any of aspects 108-116, further comprising a vent in at least one of the dilator or sheath.
Aspect 118: The access device of aspect 117, wherein the vent allows the egress of air and hinders the egress of a fluid to the ambient atmosphere.
Aspect 119: The access device of aspect 118, wherein the vent comprises a porous material.
Aspect 120: The access device of aspect 118, wherein the vent comprises a narrow channel.
Aspect 121: The access device of aspect 108, wherein the outer surface of the dilator is oblong and the inner surface of the sheath is round.
Aspect 122: The access device of aspect 108, wherein the outer surface of the dilator is round and the inner surface of the sheath is square.
Aspect 123: The access device of aspect 108, wherein the gap follows a spiral path along the longitudinal axis.
Aspect 124: The access device of aspect 108, wherein the gap follows a linear path along the longitudinal axis.
Aspect 125: The access device of aspect 124, wherein the linear path is parallel to the longitudinal axis.
Aspect 126: The access device of aspect 108, wherein a thickness of the gap varies along at least a portion of a length of the gap.
Aspect 127: The access device of aspect 108, wherein one of the medical device and the dilator include an a grooved surface.
Aspect 128: An access device for placing a medical article within a body space, comprising: a needle having an elongated needle body with a distal end and a hub from which the needle body extends, the elongated needle body comprising at least one side fenestration; a dilator disposed on the needle body, comprising a dilator hub and an elongated dilator shaft that extends from the dilator hub, wherein the dilator shaft and the elongated needle body form one or more spaces, at least one of the spaces communicating with the side fenestration in the needle; and a medical article comprising a tubular section and a hub, the tubular section being disposed on the dilator, wherein at least portions of the dilator and the medical article are configured so as to allow visual determination of the presence of a bodily fluid within the space; and wherein at least one of the needle and dilator further comprise a vent in communication with the space that allows for the escape of air from the space and inhibits the escape of the bodily fluid from the space.
Aspect 129: The access device of aspect 128, wherein at least one of the inner surface of the needle, outer surface of the needle, inner surface of the dilator, outer surface of the dilator, inner surface of the sheath, and outer surface of the sheath is coated at least partially with a surfactant or a lubricious material.
Aspect 130: The access device of aspect 129, wherein the inner surface of the sheath and/or the outer surface of the dilator is coated at least partially with a surfactant or a lubricious material.
Aspect 131: The access device of either aspect 129 or 130, wherein the inner surface of the dilator and/or the outer surface of the needle is coated at least partially with a surfactant or a lubricious material.
Aspect 132: The access device of any of aspects 129-131, wherein the outer surface of the sheath is coated at least partially with a surfactant or a lubricious material.
Aspect 133: The access device of any of aspects 129-132, wherein the inner surface of the sheath is coated at least partially with a surfactant or a lubricious material.
Aspect 134: The access device of any of aspects 129-133, wherein the outer surface of the dilator is coated at least partially with a surfactant or a lubricious material.
Aspect 135: The access device of any of aspects 129-134, wherein the inner surface of the dilator is coated at least partially with a surfactant or a lubricious material.
Aspect 136: The access device of any of aspects 129-135, wherein the outer surface of the needle is coated at least partially with a surfactant or a lubricious material.
Aspect 137: The access device of any of aspects 129-136, wherein the inner surface of the needle is coated at least partially with a surfactant or a lubricious material.
Aspect 138: The access device of aspect 128, wherein the needle body comprises one or more ridges.
Aspect 139: The access device of aspect 128, wherein the dilator shaft comprises one or more ridges.
Aspect 140: The access device of aspect 138, wherein the one or more ridges are generally straight along the needle shaft.
Aspect 141: The access device of aspect 138, wherein the one or more ridges form a channel.
Aspect 142: The access device of aspect 128 or 141, wherein the needle hub comprises one or more venting grooves.
Aspect 143: The access device of aspect 142, wherein the venting grooves comprise a porous material that permits gases to flow through the material but inhibits the passage of a body fluid.

## Claims

1. An access device for placing a medical article within a body space, comprising:
a needle having an elongated needle body with a distal end and a hub from which the needle body extends, the elongated needle body comprising at least one side fenestration;
a dilator disposed on the needle body, comprising a dilator hub and an elongated dilator shaft that extends from the dilator hub, wherein the dilator shaft and the elongated needle body form one or more spaces, at least one of the spaces communicating with the side fenestration in the needle; and
a medical article comprising a tubular section and a hub, the tubular section being disposed on the dilator,
wherein at least portions of the dilator and the medical article are configured so as to allow visual determination of the presence of a bodily fluid within the at least one space; and
wherein at least one of the needle and dilator further comprise a vent in communication with the at least one space that allows for the escape of air from the space and inhibits the escape of the bodily fluid from the space.

2. The access device of Claim 1, wherein at least one of the inner surface of the needle, outer surface of the needle, inner surface of the dilator, outer surface of die dilator, inner surface of the medical article, and outer surface of the medical article is coated at least partially with a surfactant or a lubricious material.

3. The access device of Claim 1, wherein at least one of the outer surface of the needle, inner surface of the dilator, outer surface of the dilator, and inner surface of the sheath is coated at least partially with a substance to affect capillary action within the access device.

4. The access device of Claim 1, wherein the needle body comprises one or more ridges.

5. The access device of Claim 1, wherein the dilator shaft comprises one or more ridges.

6. The access device of either of Claims 4 or 5, wherein the one or more ridges form a channel that defines the at least one space.

7. The access device of Claim 6, wherein the one or more ridges form multiple channels.

8. The access device of Claim 1 or 6, wherein at least one of the needle hub or dilator hub comprises one or more venting grooves.

9. The access device of Claim 8, wherein corresponding faces of the needle hub and dilator hub rest far enough apart to allow air to pass between them, from the venting grooves.

10. The access device of Claim 8, wherein one or more venting grooves comprise a porous material that permits gases to flow through the material but inhibits the passage of a body fluid.

11. The access device of Claim 10, wherein one or more venting grooves comprise a groove notch configured to receive the porous material.

12. The access device of Claim 8, wherein one or more venting grooves comprise a passage sufficiently small in cross-sectional area to allow the escape of gases to the ambient atmosphere while hindering the escape to the ambient atmosphere of a body fluid.

13. The access device of Claim 8, wherein one or more venting grooves are in communication with the channel.

14. The access device of Claim 8, wherein one or more venting grooves are disposed on a luer connection between the needle and dilator, and the luer connection otherwise forms a substantially liquid tight seal.

15. The access device of Claim 1, wherein corresponding faces of the needle hub and dilator hub rest far enough apart to allow air to vent between them.
